# FASCICULE DE BREVET EUROPEEN

(11) **EP 4 010 380 B1**
(45) Date de publication et mention de la délivrance du brevet: **04.10.2023**
(21) Numéro de dépôt: 20757650.5
(22) Date de dépôt: 06.08.2020
(51) Int. Cl.: C08K 5/3445

(54) **COMPOSITION A BASE D'AU MOINS UN COMPOSE AYANT UNE FONCTION IMIDAZOLIDINONE N-SUBSTITUÉE**
ZUSAMMENSETZUNG AUF BASIS VON MINDESTENS EINER VERBINDUNG MIT EINER N-SUBSTITUIERTEN IMIDAZOLIDINONFUNKTION
COMPOSITION BASED ON AT LEAST ONE COMPOUND HAVING AN N-SUBSTITUTED IMIDAZOLIDINONE FUNCTION

(30) Priorité: 07.08.2019 FR 1909030
(43) Date de publication de la demande: 15.06.2022
(73) Titulaire: COMPAGNIE GENERALE DES ETABLISSEMENTS MICHELIN, 63000 Clermont-Ferrand (FR)
(72) Inventeur: JEAN-BAPTISTE-DIT-DOMINIQUE, François, 63040 CLERMONT-FERRAND Cedex 9 (FR); FLEURY, Etienne, 63040 CLERMONT-FERRAND CEDEX 9 (FR); GANDER, Sophie, 69040 CLERMONT-FERRAND CEDEX 9 (FR)
(74) Mandataire: Bocchi, Brigitte
(86) Numéro de dépôt international: PCT/FR2020/051440
(87) Numéro de publication internationale: WO 2021/023949

(56) Documents cités:
- WO-A1-2019/007883
- WO-A1-2019/007884

## Description

La présente invention concerne une composition de caoutchouc, utilisable notamment pour la fabrication de produits pour pneumatiques, à base d'au moins un élastomère diénique, au moins une charge renforçante, au moins un système de réticulation chimique et d'au moins un composé ayant une fonction imidazolidinone N-substituée. L'invention concerne également un pneumatique comprenant une telle composition.

Dans le domaine industriel des objets fabriqués à partir de compositions de caoutchouc, notamment des pneumatiques, des mélanges de polymères avec des charges renforçantes sont souvent utilisés. Pour que de telles compositions présentent de bonnes propriétés, on recherche en permanence des moyens pour améliorer la dispersion des charges renforçantes au sein de ces polymères. En effet, une bonne dispersion de ces charges au sein de ces polymères est souvent synonyme d'une hystérèse faible et donc d'une résistance au roulement améliorée lorsque ces compositions de caoutchouc sont utilisées dans des pneumatiques.

Or, l'amélioration de la résistance au roulement est un challenge permanent pour les manufacturiers de pneumatiques depuis que les économies de carburant et la nécessité de protéger l'environnement sont devenues une priorité.

De nombreuses solutions ont été proposées pour atteindre l'objectif de baisse de l'hystérèse.

En particulier, on peut citer la modification de la structure des polymères, notamment des élastomères diéniques, en fin de polymérisation au moyen d'agents de modification, le but étant d'atteindre une bonne interaction entre le polymère ainsi modifié et la charge renforçante, qu'il s'agisse de noir de carbone ou d'une charge inorganique renforçante telle que de la silice. On peut citer par exemple le document EP0778311B1 qui décrit l'utilisation de polymères diéniques fonctionnalisés par un groupement silanol en extrémité de chaîne. Plus récemment, la demande de brevet WO2009/077837A1 décrit des élastomères fonctionnalisés par un groupement silanol à une extrémité de chaîne et par un groupement aminé à l'autre extrémité de chaîne. Ces polymères modifiés sont efficaces pour réduire l'hystérèse.

Cependant, les manufacturiers de pneumatiques cherchent toujours à réduire l'hystérèse d'une composition de caoutchouc.

C'est la raison pour laquelle, des recherches ont été menées sur d'autres réactions de modification de polymères, notamment d'élastomères diéniques, en vue de l'obtention de compositions de caoutchouc possédant une diminution de l'hystérèse. A ce titre, on peut mentionner l'utilisation de composés portant des groupements associatifs azotés comprenant au moins un motif les rendant susceptibles de s'associer entre eux par des liaisons hydrogène pour modifier les polymères utilisés dans les compositions de caoutchouc Ces polymères modifiés porteurs de fonctions imidazolidinone non-substituées sont décrits dans le document WO2019/007884A1.

Cependant, il existe chez les manufacturiers de pneumatiques un besoin permanent de toujours continuer à diminuer encore plus l'hystérèse de compositions de caoutchouc et c'est l'objectif de la présente invention.

Ce but est atteint en ce que la demanderesse a découvert, de manière surprenante, au cours de ses recherches que l'utilisation dans une composition de caoutchouc, d'un composé ayant une fonction imidazolidinone N-substituée permet de diminuer l'hystérèse de cette composition par rapport à une composition comprenant un composé ayant une fonction imidazolidinone non-substituée.

L'invention a pour objet une composition de caoutchouc à base d'au moins un élastomère diénique, d'au moins une charge renforçante, d'au moins un système de réticulation chimique et d'au moins un composé de formule (I) éventuellement déjà greffé sur ledit élastomère diénique 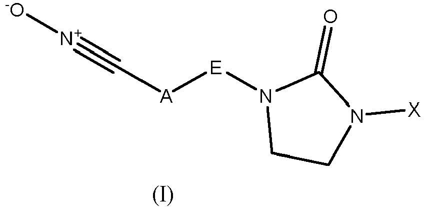 dans laquelle :
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

Un autre objet de l'invention concerne un article semi-fini en caoutchouc pour pneumatique, caractérisé en ce qu'il comprend une composition de caoutchouc telle que définie ci-dessus.

Un autre objet de l'invention concerne un pneumatique comprenant au moins un article semi-fini tel que décrit ci-dessus ou bien au moins une composition de caoutchouc telle que définie ci-dessus.

Un premier objet de l'invention concerne une composition de caoutchouc à base d'au moins un élastomère diénique, d'au moins une charge renforçante, d'au moins un système de réticulation chimique et d'au moins un composé de formule (I) éventuellement déjà greffé sur ledit élastomère diénique 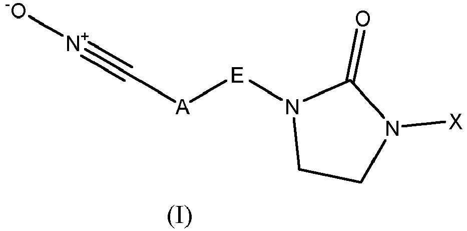 dans laquelle :
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

Par l'expression « composition à base de », il faut entendre une composition comportant le mélange et/ou le produit de réaction in situ des différents constituants utilisés, certains de ces constituants pouvant réagir et/ou étant destinés à réagir entre eux, au moins partiellement, lors des différentes phases de fabrication de la composition ; la composition pouvant ainsi être à l'état totalement ou partiellement réticulé ou à l'état non-réticulé.

Dans la présente, sauf indication expresse différente, tous les pourcentages (%) indiqués sont des pourcentages (%) en masse.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a jusqu'à b (c'est-à-dire incluant les bornes strictes a et b).

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Par « groupement associatif », on entend des groupes susceptibles de s'associer notamment les uns aux autres par des liaisons hydrogène, ioniques et/ou hydrophobes. Il s'agit, selon un mode préféré de l'invention, de groupes susceptibles de s'associer par des liaisons hydrogène. Lorsque les groupes associatifs sont susceptibles de s'associer par des liaisons hydrogène, chaque groupe associatif comporte de préférence au moins un site donneur et un site accepteur vis-à-vis de la liaison hydrogène de sorte que deux groupes associatifs identiques sont auto-complémentaires et peuvent s'associer entre eux en formant au moins deux liaisons hydrogène. Les groupes associatifs selon l'invention sont également susceptibles de s'associer par des liaisons hydrogène, ioniques et/ou hydrophobes à des fonctions présentes sur des charges, notamment sur les charges renforçantes et en particulier sur les charges renforçantes inorganiques, telles que la silice.

Par « groupement non associatif », on entend des groupes qui ne sont pas aptes à s'associer notamment les uns aux autres par des liaisons hydrogène, ioniques et/ou hydrophobes. Préférentiellement, il s'agit de groupes qui ne sont pas aptes à s'associer par des liaisons hydrogène. Ces groupes ne comportent pas de site donneur et de site accepteur vis-à-vis de la liaison hydrogène de sorte qu'aucun de ces groupes identiques ne sont auto-complémentaires. Ils ne peuvent pas s'associer entre eux en formant au moins deux liaisons hydrogène.

Par « hétéroatome », sauf mention contraire, on entend un atome choisi dans le groupe constitué par un atome de soufre, un atome d'oxygène et un atome d'azote.

### Composé de formule générale (I)

Comme expliqué précédemment, la composition de caoutchouc selon l'invention est à base d'au moins un composé de formule (I) 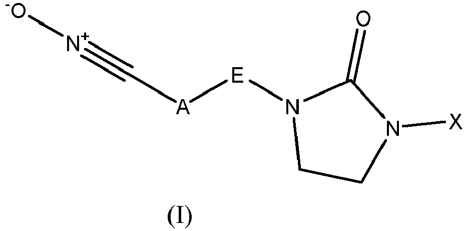 dans laquelle :
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes.

On entend au sens de la présente invention par « cycle arènediyle », un groupe hydrocarboné aromatique monocyclique ou polycyclique, dérivé d'un arène dans lequel deux atomes d'hydrogène ont été supprimés. Un cycle arènediyle est donc un groupe divalent.

Par « groupe hydrocarboné aromatique monocyclique ou polycyclique », on entend au sens de la présente invention un ou des cycles aromatiques dont le squelette est constitué d'atomes de carbone. Autrement dit, il n'y a pas d'hétéroatomes dans le squelette du cycle. Le cycle arènediyle peut être monocyclique, c'est-à-dire constitué d'un seul cycle, ou polycyclique, c'est-à-dire constitué de plusieurs cycles hydrocarbures aromatiques condensés ; de tels cycles condensés ont alors en commun au moins deux atomes de carbone successifs. Ces cycles peuvent être ortho-condensés ou ortho- et péri-condensés.

De préférence, le cycle arènediyle comprend de 6 à 14 atomes de carbone.

Le cycle arènediyle peut être non substitué, partiellement substitué ou totalement substitué. Un cycle arènediyle est partiellement substitué lorsqu'un ou deux ou plusieurs atomes d'hydrogène (mais pas tous les atomes) sont remplacés par une ou deux ou plusieurs chaînes hydrocarbonées, aliphatiques, de préférence saturées, linéaires ou ramifiées, éventuellement substituées par un ou plusieurs hétéroatomes. Lesdites chaînes sont aussi appelées substituants. Si tous les atomes d'hydrogène sont remplacés par lesdites chaînes, alors le cycle arènediyle est totalement substitué. Les substituants du cycle arènediyle peuvent être identiques ou différents les uns par rapport aux autres.

De préférence, lorsque le cycle arènediyle, est substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes, cette ou ces chaînes peuvent être inertes vis-à-vis de la fonction imidazolidinone N-substituée et de l'oxyde de nitrile.

On entend, au sens de la présente invention, par « chaîne hydrocarbonée inerte vis-à-vis de la fonction imidazolidinone N-substituée et de l'oxyde de nitrile » une chaîne hydrocarbonée qui ne réagit ni avec ladite fonction imidazolidinone N-substituée ni avec ledit oxyde de nitrile. Ainsi, ladite chaîne hydrocarbonée inerte par rapport à ladite fonction imidazolidinone N-substituée et audit oxyde de nitrile est, de préférence, une chaîne hydrocarbonée aliphatique qui ne présente pas de fonctions alcényle ou alcynyle, susceptibles de réagir avec ladite fonction ou ledit groupement, c'est-à-dire est une chaîne hydrocarbonée aliphatique saturée, linéaire ou ramifiée, éventuellement substituée ou interrompue par un ou plusieurs hétéroatomes, et comprenant préférentiellement de 1 à 24 atomes de carbone.

De préférence, le groupement A est un cycle arènediyle en C₆-C₁₄ éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes. Plus préférentiellement, le groupement A est un cycle arènediyle en C₆-C₁₄, éventuellement substitué par une ou plusieurs chaînes hydrocarbonées, identiques ou différentes, saturées, en C₁-C₂₄, linéaires ou ramifiées, éventuellement substituées ou interrompues par un ou plusieurs hétéroatomes d'azote, de soufre ou d'oxygène. Plus préférentiellement encore, le groupement A est un cycle arènediyle en C₆-C₁₄, éventuellement substitué par un ou plusieurs substituants, identiques ou différents, choisis dans le groupe constitué par un alkyle en C₁-C₁₂, (plus préférentiellement en C₁-C₆, plus préférentiellement encore en C₁-C₄), un groupement OR', un groupement -NHR', un groupement -SR' où R' est un groupe alkyle en C₁-C₁₂, plus préférentiellement en C₁-C₆, plus préférentiellement encore en C₁-C₄.

De préférence, le composé de formule (I) est choisi parmi les composés de formule (Ia) et (Ib) 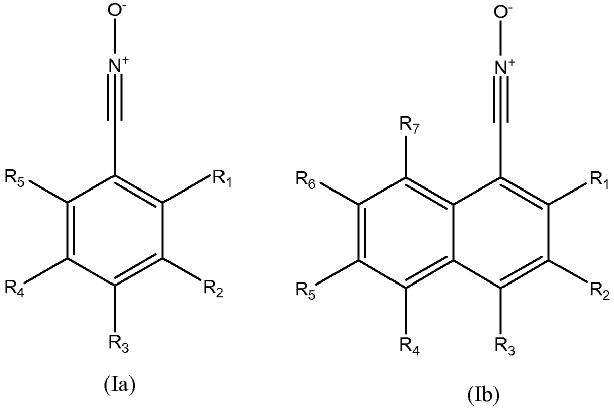 dans lesquelles :
- un groupement choisi parmi R₁ à R₅ de la formule (Ia) et un groupement choisi parmi R₁ à R₇ de la formule (Ib) désignent le groupe de formule (II) suivante : 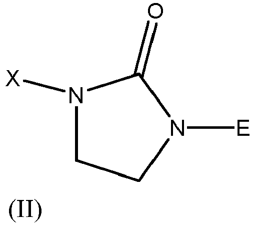 dans laquelle E et X sont tels que définis précédemment,
- les quatre groupements de la formule (Ia) choisis parmi R₁ à R₅ autres que celui désignant le groupe de formule (II) et les six groupements de la formule (Ib) choisis parmi R₁ à R₇ autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

Préférentiellement, dans les composés de formule (Ia) et (Ib), les quatre groupements de la formule (Ia) choisis parmi R₁ à R₅ autres que celui désignant le groupe de formule (II) et les six groupements de la formule (Ib) choisis parmi R₁ à R₇ autres que celui désignant le groupe de formule (II), identiques ou différents, représentent indépendamment les uns des autres, un atome d'hydrogène ou une chaîne hydrocarbonée aliphatique, saturée, linéaire ou ramifiée, en C₁-C₂₄, éventuellement substituée ou interrompue par un ou plusieurs hétéroatomes.

Plus préférentiellement encore, dans les composés de formule (Ia) et (Ib), les quatre groupements de la formule (Ia) choisis parmi R₁ à R₅ autres que celui désignant le groupe de formule (II) et les six groupements de la formule (Ib) choisis parmi R₁ à R₇ autres que celui désignant le groupe de formule (II), identiques ou différents, sont choisis dans le groupe formé par un atome d'hydrogène, un alkyle en C₁-C₁₂ (plus préférentiellement en C₁-C₆, plus préférentiellement encore en C₁-C₄), un groupement-OR', un groupement -NHR' et un groupement -SR' où R' est un alkyle en C₁-C₁₂, plus préférentiellement en C₁-C₆, plus préférentiellement encore en C₁-C₄.

Selon un mode de réalisation préféré de l'invention, dans la formule (Ia), R₂ représente un groupe de formule (II) tel que défini ci-dessus et R₁, R₃, R₄ et R₅, identiques ou différents, représentent un atome d'hydrogène ou une chaîne hydrocarbonée aliphatique, de préférence saturée, linéaire ou ramifiée, en C1-C₂₄, éventuellement substituée ou interrompue par un ou plusieurs hétéroatomes. Plus préférentiellement, R₂ représente un groupe de formule (II) tel que défini ci-dessus et R₁, R₃, R₄ et R₅, identiques ou différents, sont choisis dans le groupe constitué par un atome d'hydrogène, un alkyle en C₁-C₁₂ (plus préférentiellement en C₁-C₆, plus préférentiellement encore en C₁-C₄), un groupement-OR', un groupement -NHR' et un groupement -SR' où R' est un alkyle en C₁-C12, plus préférentiellement en C₁-C₆, plus préférentiellement encore en C₁-C₄.

Plus préférentiellement encore dans ce mode de réalisation, R₂ représente un groupe de formule (II) tel que défini ci-dessus, R₄ représente un atome d'hydrogène et R₁ R₃ et R₅ représentent une chaîne hydrocarbonée aliphatique, de préférence saturée, linéaire ou ramifiée, en C₁-C₂₄, éventuellement substituée ou interrompue par un ou plusieurs hétéroatomes. Plus préférentiellement encore, R₂ représente un groupe de formule (II) tel que défini ci-dessus, R₄ représente un atome d'hydrogène et R₁ R₃ et R₅, identiques ou différents, sont choisis dans le groupe constitué par un alkyle en C₁-C₁₂ (plus préférentiellement en C₁-C₆, plus préférentiellement encore en C₁-C₄), un groupement-OR', un groupement -NHR' et un groupement -SR' où R' est un alkyle en C₁-C₁₂, plus préférentiellement en C₁-C₆, plus préférentiellement encore en C₁-C₄.

Selon un autre mode de réalisation préférée de l'invention, dans la formule (Ib), R₁ représente un groupe de formule (II) tel que défini ci-dessus et R₂ à R₇ identiques ou différents, représentent un atome d'hydrogène ou une chaîne hydrocarbonée aliphatique, de préférence saturée, linéaire ou ramifiée, en C₁-C₂₄, éventuellement substituée ou interrompue par un ou plusieurs hétéroatomes. Plus préférentiellement, R₁ représente un groupe de formule (II) tel que défini ci-dessus et R₂ à R₇, identiques ou différents, sont choisis dans le groupe constitué par un atome d'hydrogène, un alkyle en C₁-C₁₂ (plus préférentiellement en C₁-C₆, plus préférentiellement encore en C₁-C₄), un groupement -OR', un groupement -NHR' et un groupement -SR' où R' est un alkyle en C₁-C₁₂, plus préférentiellement en C₁-C₆, plus préférentiellement encore en C₁-C₄. Plus préférentiellement encore dans ce mode de réalisation, R₁ représente un groupe de formule (II) tel que défini ci-dessus et R₂ à R₇, identiques, représentent un atome d'hydrogène.

Dans les composés de formule (I), (Ia) et (Ib), le groupement E est un groupe divalent hydrocarboné pouvant éventuellement contenir un ou plusieurs hétéroatome(s). Par « groupe divalent hydrocarboné » on entend au sens de la présente invention, un groupe espaceur (ou un groupe de liaison) formant un pont entre le groupement A et le groupe imidazolidinone N-substitué, ce groupe espaceur étant une chaîne hydrocarbonée, saturée ou insaturée, de préférence saturée, en C₁-C₂₄, linéaire ou ramifiée, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec l'oxyde de nitrile et le groupe imidazolidinone N-substitué tel que défini ci-dessus.

Préférentiellement, dans les composés de formule (I), (Ia) et (Ib), le groupement E est une chaîne hydrocarbonée, linéaire ou ramifiée, de préférence saturée, en C₁-C₂₄, plus préférentiellement en C₁-C₁₀, encore plus préférentiellement en C₁-C₆, éventuellement interrompue par un ou plusieurs atomes d'azote, de soufre ou d'oxygène.

De préférence, dans les composés de formule (I), (Ia) et (Ib), le groupement E est choisi dans le groupe constitué par -R-, -NH-R-, -O-R- et -S-R- où R est un alkylène, linéaire ou ramifié, en C₁-C₂₄, de préférence en C₁-C₁₀, plus préférentiellement en C₁-C₆.

Plus préférentiellement encore, dans les composés de formule (I), (Ia) et (Ib), le groupement E est choisi dans le groupe constitué par -R- et -OR- où R est un alkylène, linéaire ou ramifié, en C₁-C₂₄, de préférence en C₁-C₁₀, plus préférentiellement en C₁-C₆.

Plus préférentiellement encore, dans les composés de formule (I), (Ia) et (Ib), le groupement E est choisi parmi -CH₂-, -CH₂-CH₂-, -CH₂-CH₂-CH₂-, -CH₂-CH₂-CH₂-CH₂-, -O-CH₂-, -O-CH2-CH₂-, -O-CH₂-CH₂-CH₂- et -O-CH₂-CH₂-CH₂-CH₂-.

Selon un mode de réalisation préférée, le composé de formule (I) est le composé de formule (Ib).

Selon ce mode de réalisation préféré, on préfère particulièrement le composé de formule (Ib) tel que défini ci-dessus avec le groupement E choisi dans le groupe constitué par -R- et -OR- où R est un alkylène, linéaire ou ramifié, en C₁-C₂₄, de préférence en C₁-C₁₀, plus préférentiellement en C₁-C₆ et avec le groupement X qui est un atome de chlore.

Selon ce mode de réalisation, plus préférentiellement encore, le composé de formule (I) est le composé de formule (Ib) avec :
- R₁ représente un groupe de formule (II) dans lequel le groupement E est choisi dans le groupe constitué par -R- et -OR- où R est un alkylène, linéaire ou ramifié, en C₁-C₂₄, de préférence en C₁-C₁₀, plus préférentiellement en C₁-C₆ et le groupement X désigne un atome de chlore ; et
- R₂ à R₇ identiques ou différents, représentent un atome d'hydrogène ou une chaîne hydrocarbonée aliphatique, de préférence saturée, linéaire ou ramifiée, en C₁-C₂₄, éventuellement substituée ou interrompue par un ou plusieurs hétéroatomes.

Selon ce mode de réalisation, plus préférentiellement encore, le composé de formule (I) est le composé de formule (Ib) avec :
- R₁ représente un groupe de formule (II) dans lequel le groupement E est choisi dans le groupe constitué par -R- et -OR- où R est un alkylène, linéaire ou ramifié, en C₁-C₂₄, de préférence en C₁-C₁₀, plus préférentiellement en C₁-C₆ et le groupement X désigne un atome de chlore ; et
- R₂ à R₇, identiques ou différents, sont choisis dans le groupe constitué par un atome d'hydrogène, un alkyle en C₁-C₁₂ (plus préférentiellement en C₁-C₆, plus préférentiellement encore en C₁-C₄), un groupement -OR', un groupement -NHR' et un groupement -SR' où R' est un alkyle en C₁-C₁₂, plus préférentiellement en C₁-C₆, plus préférentiellement encore en C₁-C₄.

Selon ce mode de réalisation, plus préférentiellement encore, le composé de formule (I) est le composé de formule (Ib) avec :
- R₁ représente un groupe de formule (II) dans lequel le groupement E est choisi dans le groupe constitué par -R- et -OR- où R est un alkylène, linéaire ou ramifié, en C₁-C₂₄, de préférence en C₁-C₁₀, plus préférentiellement en C₁-C₆ et le groupement X désigne un atome de chlore ; et
- R₂ à R₇, identiques, représentent un atome d'hydrogène.

Selon un mode de réalisation particulier, le composé de formule (I) est choisi dans le groupe constitué par le composé de formule (III) et de formule (IV), plus préférentiellement est le composé de formule (III)

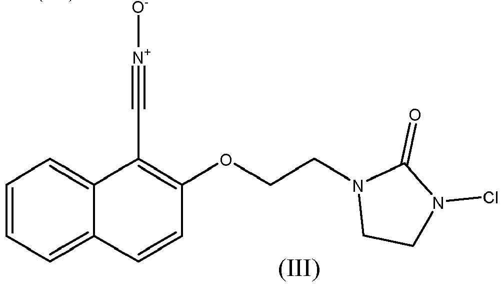

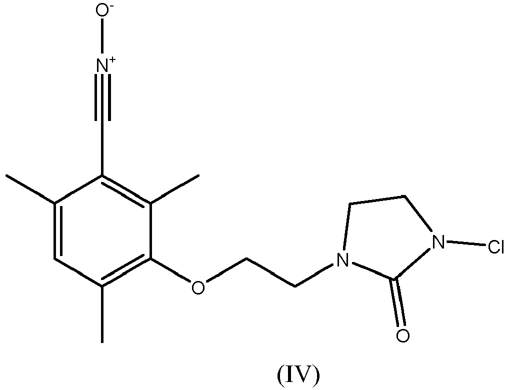

Les compositions de caoutchouc selon l'invention comprenant au moins un composé ayant un groupement imidazolidinone N-substitué, c'est-à-dire un groupement azoté non associatif, de formule (I), de préférence un composé de formule (Ib), et plus préférentiellement encore un composé de formule (III), éventuellement déjà greffé sur l'élastomère diénique, présentent, de manière surprenante, une diminution significative de leur hystérèse, c'est-à-dire une amélioration de la performance au roulement.

Les composés de formule (I), en particulier ceux de formule (Ia), (Ib), (III) et (IV) peuvent être obtenu à partir d'un procédé de synthèse comprenant les étapes successives suivantes :
(b1) la réaction d'un composé de formule (IX) suivante : 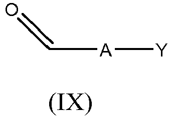 dans laquelle A est tel que défini précédemment et Y représente un groupe nucléophile, avec un composé de formule (X) suivante : 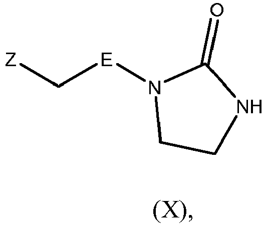 dans laquelle E est tel que défini précédemment, et Z représente un groupe nucléofuge ;
   en présence d'au moins un solvant polaire S1, d'au moins une base, à une température T1 allant de 70 à 150°C, pour former un composé de formule (XI) suivante : 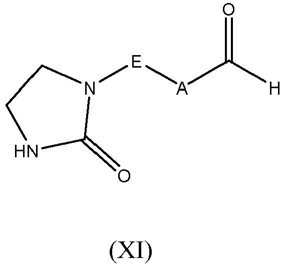
(b2) la réaction dudit composé de formule (XI) avec une solution aqueuse d'hydroxylamine à une température T2 allant de 30 à 70°C, pour obtenir un composé oxime de formule (XII) suivante : 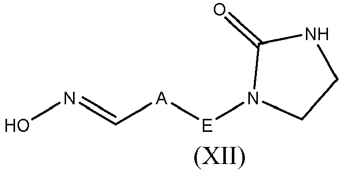
(c) une étape de récupération dudit composé oxime de formule (XII) ;
(d) une étape d'oxydation du composé oxime de formule (XII) avec un agent oxydant, en présence d'au moins un solvant organique S2, le taux de l'agent oxydant étant d'au moins 6 équivalents molaire par rapport à la quantité molaire de composé oxime de formule (XII).

On entend au sens de la présente demande par « solvant polaire » un solvant présentant une constante diélectrique supérieure à 2,2.

On entend au sens de la présente demande « par groupe nucléofuge » un groupe partant qui emporte son doublet de liaison.

On entend au sens de la présente demande « par groupe nucléophile » un composé comprenant au moins un atome porteur d'un doublet libre ou un atome chargé négativement.

Comme expliqué précédemment, le procédé de synthèse du composé de formule (I) comprend notamment les étapes successives (b1) et (b2).

Selon un mode de réalisation particulier, les deux étapes (b1) et (b2) sont séparées par une étape d'isolation et de purification du composé de formule (XI).

Selon un autre mode de réalisation, les deux étapes (b1) et (b2) sont réalisées selon une synthèse monotope, c'est-à-dire que les étapes (b1) et (b2) sont « one pot » (procédé de synthèse monotope en deux étapes), soit sans isolation du composé de formule (XI) intermédiaire.

Le procédé comprend une étape (b1) de réaction d'un composé de formule (IX), telle que mentionnée ci-dessus, porteur d'un groupe Y, avec un composé de formule (X), telle que mentionnée ci-dessus, porteur d'un groupe Z.

De manière préférée, le groupe Y est choisi parmi les fonctions hydroxyle, thiol et amine primaire ou secondaire.

Le groupe Z peut être choisi parmi le chlore, le brome, l'iode, le groupe mésylate, le groupe tosylate, le groupe acétate et le groupe trifluorométhylsulfonate.

Ladite étape (b1) du procédé est réalisée en présence d'au moins un solvant polaire S1, et d'au moins une base, à une température T1 allant de 70 à 150°C.

Selon un mode de réalisation particulier du procédé, le solvant polaire S1 est un solvant polaire miscible dans l'eau, préférentiellement un solvant protique.

Le diméthylformamide (DMF), le diméthylsulfoxyde (DMSO), la 1,3-diméthyl-2-imidazolidinone (DMI), la 1,3-diméthyl-3,4,5,6-tetrahydro-2(1H)-pyrimidinone (DMPU), l'isopropanol, l'acétonitrile, l'éthanol, le n-butanol et le n-propanol sont des exemples de solvants S1 utilisables dans le procédé.

De préférence, le solvant protique est alcoolique.

Avantageusement, le composé de formule (IX) représente de 5 à 40% en poids, de préférence de 10 à 30% en poids, par rapport au poids du solvant.

La base peut être choisie parmi les alcoolates alcalins, les carbonates alcalins, les carbonates alcalino-terreux, les hydroxydes alcalins, les hydroxydes alcalino-terreux et leurs mélanges.

Avantageusement, il est possible d'ajouter :
- un ou plusieurs catalyseurs choisis parmi un catalyseur de type sel d'argent (I), un catalyseur de transfert de phase de type ammonium quaternaire, et leurs mélanges ;
- un ou plusieurs liquides ioniques.

Préférentiellement, la base est choisie parmi le méthanolate de sodium, le carbonate de potassium et la soude, plus préférentiellement le carbonate de potassium.

Selon un mode de réalisation particulier du procédé, la quantité molaire de base est de 1,5 à 8 équivalents molaire, de préférence de 2 à 6 équivalents molaire, par rapport à la quantité molaire de composé de formule (IX).

Comme expliqué précédemment, l'étape (b1) du procédé est réalisée à une température T1 allant de 70 à 150°C.

De préférence, la température T1 est une température allant de 70 à 120°C, plus préférentiellement de 80 à 110°C.

Comme expliqué précédemment, l'étape (b1) du procédé est suivie de l'étape (b2) de l'ajout dans le milieu réactionnel contenant le composé de formule (XI) d'une solution aqueuse d'hydroxylamine à une température T2 allant de 30 à 70°C.

Préférentiellement, l'ajout de la solution aqueuse d'hydroxylamine est réalisé lorsque la conversion du composé de formule (IX) est d'au moins 70% en poids.

Avantageusement, la température T2 varie de 40 à 60°C.

Le procédé comprend également une étape (c) de récupération, telle que mentionnée ci-avant, du composé oxime de formule (XII).

De manière préférée, le composé oxime de formule (XII) est récupéré par précipitation à l'eau, suivie éventuellement d'un lavage à l'eau.

Le procédé comprend également une étape (d) d'oxydation du composé oxime de formule (XII) avec un agent oxydant pour donner le composé de formule (I), en particulier les composés préférés, en présence d'au moins un solvant organique S2 ; la quantité d'agent oxydant est d'au moins 6 équivalents molaire, préférentiellement de 6,5 à 15 équivalents molaire, par rapport à la quantité molaire de composé oxime de formule (XII).

Cette quantité d'agent oxydant peut être ajoutée en une fois ou en plusieurs fois au cours de l'étape (d), de préférence ajoutée en deux fois au cours de l'étape (d).

De manière préférée, ledit agent oxydant est choisi parmi l'hypochlorite de sodium, le N-bromosuccinimide en présence d'une base et le N-chlorosuccinimide en présence d'une base, préférentiellement ledit agent oxydant est l'hypochlorite de sodium.

Préférentiellement, le solvant organique S2 est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

De préférence, le composé oxime de formule (XII) représente de 1 à 30% en poids, de préférence de 1 à 20% en poids, par rapport au poids total de l'ensemble comprenant ledit composé oxime de formule (XII), ledit solvant organique S2 et ledit agent oxydant. Préférentiellement, le procédé comprend, après l'étape (d), une étape (e) de récupération du composé de formule (I).

Selon un mode de réalisation particulier, le procédé peut comprendre une étape (a2) de fabrication du composé de formule (X), préalable à l'étape (b1), par mise en réaction d'un composé de formule (XIII) suivante : 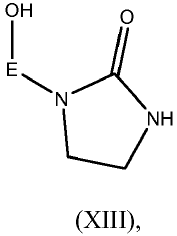 dans laquelle :
- E est tel que défini précédemment,
- avec un agent permettant la formation du groupe nucléofuge Z.

De manière préférée, ledit agent est le chlorure de thionyle.

De préférence, l'étape (a2) est réalisée en l'absence ou en présence d'au moins un solvant S4, préférentiellement un solvant chloré, plus préférentiellement le dichlorométhane.

Avantageusement, l'étape (a2) est immédiatement suivie d'une étape (a3) de récupération du composé de formule (X), préférentiellement par purification au toluène, plus préférentiellement par cristallisation du composé de formule (X) dans le toluène.

Préférentiellement, le taux molaire de composé de formule (I) dans la composition de caoutchouc selon l'invention, est compris dans un domaine allant de 0,01 à 5 % molaire, de préférence de 0,01 à 1 % molaire, plus préférentiellement de 0,1 à 1 % molaire, qu'il s'agisse indifféremment de motifs diéniques ou non diéniques.

On entend, par « taux de composé de formule (I) » présent dans une composition de caoutchouc, exprimé en pourcentage molaire, le nombre de de composés de formule (I) présents dans la composition de caoutchouc pour cent motifs d'élastomère diénique de la composition. Par exemple, si le taux de composé de formule (I) sur un SBR (caoutchouc styrène butadiène) est de 0,20 % molaire, cela signifie qu'il y aura 0,20 motif issu de composé de formule (I) pour 100 motifs styrèniques et butadiéniques de SBR.

Dans le cas où on utilise dans la composition à la fois un élastomère déjà greffé par le composé de formule (I) et un élastomère diénique non greffé par le composé de formule (I), le taux de composé de formule (I) représente le nombre de composés de formule (I) greffés pour 100 motifs d'élastomères diéniques, le nombre de motifs prenant en compte les deux élastomères (greffé et non greffé), en supposant que d'autres composés de formule (I) non déjà greffés n'ont pas été ajoutés dans la composition.

### Élastomère

Comme expliqué précédemment, la composition de caoutchouc selon l'invention est à base d'au moins un élastomère diénique.

Par élastomère (ou indistinctement caoutchouc) « diénique », qu'il soit naturel ou synthétique, doit être compris de manière connue un élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : « essentiellement insaturés » ou « essentiellement saturés ». On entend en général par « essentiellement insaturé », un élastomère diénique issu au moins en partie de monomères diènes conjugués, ayant un taux de motifs ou unités d'origine diénique (diènes conjugués) qui est supérieur à 15 % (% en moles); c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques « essentiellement saturés » (taux de motifs d'origine diénique faible ou très faible, toujours inférieur à 15 %).

On entend particulièrement par élastomère diénique susceptible d'être utilisé dans le cadre de la présente invention :
- tout homopolymère d'un monomère diène, conjugué ou non, ayant de 4 à 18 atomes de carbone ;
- tout copolymère d'un diène, conjugué ou non, ayant de 4 à 18 atomes de carbone et d'au moins un autre monomère.

L'autre monomère peut être l'éthylène, une oléfine ou un diène, conjugué ou non.

À titre de diènes conjugués conviennent les diènes conjugués ayant de 4 à 12 atomes de carbone, en particulier les 1,3-diènes, tels que notamment le 1,3-butadiène et l'isoprène.

À titre de diènes non conjugués conviennent les diènes non conjugués ayant de 6 à 12 atomes de carbone, tels que le 1,4-hexadiène, l'éthylidène norbomène, le dicyclopentadiène.

À titre d'oléfines conviennent les composés vinylaromatiques ayant de 8 à 20 atomes de carbone et les α-monooléfmes aliphatiques ayant de 3 à 12 atomes de carbone.

À titre de composés vinylaromatiques conviennent par exemple le styrène, l'ortho-, méta-, para-méthylstyrène, le mélange commercial « vinyle-toluène », le para-tertiobutylstyrène.

À titre d'α-monooléfines aliphatiques conviennent notamment les α-monooléfines aliphatiques acycliques ayant de 3 à 18 atomes de carbone.

Plus particulièrement, l'élastomère diénique peut être :
- tout homopolymère d'un monomère diène conjugué, notamment tout homopolymère obtenu par polymérisation d'un monomère diène conjugué ayant de 4 à 12 atomes de carbone;
- tout copolymère obtenu par copolymérisation d'un ou plusieurs diènes conjugués entre eux ou avec un ou plusieurs composés vinylaromatiques ayant de 8 à 20 atomes de carbone;
- un copolymère d'isobutène et d'isoprène (caoutchouc butyle), ainsi que les versions halogénées, en particulier chlorées ou bromées, de ce type de copolymère ;
- tout copolymère obtenu par copolymérisation d'un ou plusieurs diènes, conjugués ou non, avec l'éthylène, une α-monooléflne ou leur mélange comme par exemple les élastomères obtenus à partir d'éthylène, de propylène avec un monomère diène non conjugué du type précité.

Préférentiellement, l'élastomère diénique est choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène, les copolymères d'isoprène et les mélanges de ces élastomères.

Préférentiellement, l'élastomère diénique est choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène-styrène (SBR), les copolymères d'éthylène-butadiène (EBR), les copolymères d'isoprène-butadiène (BIR) ou les copolymères d'isoprène-butadiène-styrène (SBIR), les copolymères d'isobutène-isoprène (caoutchouc butyle - IIR), les copolymères d'isoprène-styrène (SIR) et les mélanges de ces élastomères.

Préférentiellement, l'élastomère diénique est choisi dans le groupe constitué par les copolymères d'éthylène-propylène-monomère diène, le caoutchouc butyle et le mélange de ces caoutchoucs.

Préférentiellement, l'élastomère diénique est choisi dans le groupe constitué par le caoutchouc naturel, les polyisoprènes de synthèse, les polybutadiènes, les copolymères de butadiène, les copolymères d'isoprène et les mélanges de ces élastomères.

Plus préférentiellement, l'élastomère diénique est choisi dans le groupe constitué par le caoutchouc naturel, les polyisoprènes de synthèse, les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Préférentiellement, l'élastomère diénique est choisi dans le groupe constitué par les polybutadiènes, les copolymères de butadiène, les copolymères d'isoprène et les mélanges de ces élastomères.

Plus préférentiellement, l'élastomère diénique est choisi dans le groupe constitué par les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse (Tg, mesurée selon ASTM D3418 (2015)) comprise entre 0°C et - 90°C et plus particulièrement entre - 10°C et - 70°C, une teneur en styrène comprise entre 1% et 60% en poids et plus particulièrement entre 20% et 50%, une teneur (% molaire) en liaisons -1,2 de la partie butadiènique comprise entre 4% et 75%, une teneur (% molaire) en liaisons trans-1,4 comprise entre 10% et 80%, les copolymères de butadiène-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5 % et 50 % en poids et une Tg comprise entre - 5 C et - 50°C. Dans le cas des copolymères de butadiène-styrène-isoprène conviennent notamment ceux ayant une teneur en styrène comprise entre 5 % et 50 % en poids et plus particulièrement comprise entre 10 % et 40 %, une teneur en isoprène comprise entre 15 % et 60 % en poids et plus particulièrement entre 20 % et 50 %, une teneur en butadiène comprise entre 5 % et 50 % en poids et plus particulièrement comprise entre 20 % et 40 %, une teneur (% molaire) en unités
-1,2 de la partie butadiènique comprise entre 4 % et 85 %, une teneur (% molaire) en unités trans -1,4 de la partie butadiènique comprise entre 6 % et 80 %, une teneur (% molaire) en unités -1,2 plus -3,4 de la partie isoprènique comprise entre 5 % et 70 % et une teneur (% molaire) en unités trans -1,4 de la partie isoprènique comprise entre 10 % et 50 %, et plus généralement tout copolymère butadiène-styrène-isoprène ayant une Tg comprise entre - 5°C et - 70°C.

Les élastomères diéniques peuvent avoir toute microstructure qui est fonction des conditions de polymérisation utilisées. Ces polymères, peuvent être par exemple à blocs, statistiques, séquencés, microséquencés, et être préparer en dispersion en émulsion ou en solution. Ils peuvent être couplés et/ou étoilés, par exemple au moyen d'un atome silicium ou d'étain qui lie entre elles les chaînes polymères.

Dans un premier mode de réalisation préféré de l'invention, la composition de caoutchouc selon l'invention est à base d'au moins un élastomère diénique et au moins un composé formule (I) telle que défini précédemment, et qui est déjà greffé sur l'élastomère diénique.

Par « élastomère diénique déjà greffé par au moins un composé de formule (I) », on entend un élastomère diénique comportant des fonctions, notamment des fonctions imidazolidinone N-substituées, qui ont été introduites dans la chaîne de l'élastomère diénique. En pratique, l'élastomère diénique modifié est obtenu par réaction de greffage d'au moins un composé de formule (I) portant des fonctions N-imidazolidinone substituées et portant une fonction apte à former une liaison covalente avec une insaturation de la chaîne de l'élastomère diénique initial, cette fonction étant un oxyde de nitrile. Lors de la réaction de greffage, la ou les fonctions oxyde de nitrile du composé à greffer forment des liaisons covalentes avec la ou les insaturations de la chaîne de l'élastomère diénique.

Par « insaturation » on entend une liaison covalente multiple entre deux atomes de carbone ; cette liaison covalente multiple pouvant être une double liaison carbone-carbone ou une triple liaison carbone-carbone, de préférence une double liaison carbone-carbone.

Par « chaîne de l'élastomère diénique initial », on entend au sens de la présente invention la chaîne de l'élastomère diénique avant la réaction de greffage ; cette chaîne comprenant au moins une insaturation susceptible, plus préférentiellement au moins deux insaturations susceptibles, de réagir avec les composés ayant une fonction oxyde de nitrile. L'élastomère diénique initial est donc l'élastomère diénique servant de réactif de départ lors de la réaction de greffage. La réaction de greffage permet à partir d'un élastomère initial d'obtenir un élastomère diénique modifié (aussi appelé élastomère diénique déjà greffé).

Le procédé d'obtention de l'élastomère diénique déjà greffé par le composé de formule (I), de préférence le composé de formule (Ib), plus préférentiellement le composé de formule (III) est expliqué ci-après dans la description.

Selon un deuxième mode de réalisation préféré de l'invention, la composition de caoutchouc selon l'invention est à base d'au moins un élastomère diénique et au moins un composé de formule (I) telle que défini précédemment.

La composition de caoutchouc selon l'invention peut donc contenir un seul élastomère diénique greffé par le composé de formule (I) (soit greffé préalablement à son introduction dans la composition, soit greffé par réaction avec le composé de formule (I) pendant la fabrication de la composition) ou un mélange de plusieurs élastomères diéniques tous, greffés ou dont certains sont greffés et les autres pas.

Le ou les autres élastomères diéniques utilisés en coupage avec l'élastomère diénique de la composition de l'invention, éventuellement déjà greffé par au moins un composé de formule (I), sont des élastomères diéniques conventionnels tels que décrits plus haut qu'ils soient étoilés, couplés, fonctionnalisés ou non.

L'élastomère diénique de la composition de l'invention, éventuellement déjà greffé par au moins un composé de formule (I) peut être utilisés en association avec tout type d'élastomère synthétique autre que diénique, voire avec des polymères autres que des élastomères.

### Charge renforçante

Comme précisé précédemment, la composition de caoutchouc selon l'invention est à base d'au moins une charge renforçante.

La charge renforçante peut être de tout type de charge dite renforçante, connue pour ses capacités à renforcer une composition de caoutchouc utilisable notamment pour la fabrication de pneumatiques, par exemple une charge organique telle que du noir de carbone, une charge inorganique telle que de la silice ou encore un mélange de ces deux types de charges.

Avantageusement, la charge renforçante est choisie parmi un noir de carbone, une charge inorganique renforçante et un mélange d'un noir de carbone et d'une charge inorganique.

Comme noirs de carbone conviennent tous les noirs de carbone, notamment les noirs conventionnellement utilisés dans les pneumatiques ou leurs bandes de roulement. Parmi ces derniers, on citera plus particulièrement les noirs de carbone renforçants des séries 100, 200, 300, ou les noirs de série 500, 600 ou 700 (grades ASTM D-1765-2017), comme par exemple les noirs N115, N134, N234, N326, N330, N339, N347, N375, N550, N683, N772). Ces noirs de carbone peuvent être utilisés à l'état isolé, tels que disponibles commercialement, ou sous tout autre forme, par exemple comme support de certains des additifs de caoutchouterie utilisés. Les noirs de carbone pourraient être par exemple déjà incorporés à l'élastomère diénique, notamment isoprènique sous la forme d'un masterbatch (voir par exemple demandes WO97/36724-A2 ou WO99/16600-A1).

Comme exemple de charges organiques autres que des noirs de carbone, on peut citer les charges organiques de polyvinyle fonctionnalisé telles que décrites dans les demandes WO2006/069792-A1, WO2006/069793-A1, WO2008/003434-A1 et WO2008/003435-A1.

Par « charge inorganique renforçante », doit être entendu ici toute charge inorganique ou minérale, quelles que soient sa couleur et son origine (naturelle ou de synthèse), encore appelée charge « blanche », charge « claire » ou même charge « non-noire » par opposition au noir de carbone, capable de renforcer à elle seule, sans autre moyen qu'un agent de couplage intermédiaire, une composition de caoutchouc destinée à la fabrication de pneumatiques. De manière connue, certaines charges inorganiques renforçantes peuvent se caractériser notamment par la présence de groupes hydroxyle (-OH) à leur surface.

Comme charges inorganiques renforçantes conviennent notamment des charges minérales du type siliceux, préférentiellement la silice (SiO₂) ou du type alumineux, en particulier l'alumine (Al₂O₃).

De préférence, la charge inorganique renforçante est une silice.

La silice utilisée peut être toute silice renforçante connue de l'homme du métier, notamment toute silice précipitée ou pyrogénée présentant une surface spécifique BET ainsi qu'une surface spécifique CTAB toutes deux inférieures à 450 m²/g, de préférence comprises dans un domaine allant de 30 à 400 m²/g, notamment de 60 à 300 m²/g.

On peut utiliser tout type de silice précipitée, notamment des silices précipitées hautement dispersibles (dites « HDS » pour « highly dispersible » ou « highly dispersible silica »). Ces silices précipitées, hautement dispersibles ou non, sont bien connues de l'homme du métier. On peut citer, par exemple, les silices décrites dans les demandes WO03/016215-A1 et WO03/016387-A1. Parmi les silices HDS commerciales, on peut notamment utiliser les silices « Ultrasil ^{®} 5000GR », « Ultrasil ^{®} 7000GR » de la société Evonik, les silices « Zeosil ^{®} 1085GR», « Zeosil^{®} 1115 MP », « Zeosil^{®} 1165MP », « Zeosil^{®} Premium 200MP », « Zeosil^{®} HRS 1200 MP » de la Société Solvay. À titre de silice non HDS, les silices commerciales suivantes peuvent être utilisées : les silices « Ultrasil ^{®} VN2GR », « Ultrasil ^{®} VN3GR » de la société Evonik, la silice « Zeosil^{®} 175GR » » de la société Solvay, les silices « Hi-Sil EZ120G(-D) », « Hi-Sil EZ160G(-D) », « Hi-Sil EZ200G(-D) », « Hi-Sil 243LD », « Hi-Sil 210 », « Hi-Sil HDP 320G » de la société PPG.

L'état physique sous lequel se présente la charge inorganique renforçante est indifférent, que ce soit sous forme de poudre, de microperles, de granulés, ou encore de billes ou toute autre forme densifiée appropriée. Bien entendu on entend également par charge inorganique renforçante des mélanges de différentes charges inorganiques renforçantes, en particulier de silices telles que décrites ci-dessus.

L'homme du métier comprendra qu'en remplacement de la charge inorganique renforçante décrite ci-dessus, pourrait être utilisée une charge renforçante d'une autre nature, dès lors que cette charge renforçante d'une autre nature serait recouverte d'une couche inorganique telle que de la silice, ou bien comporterait à sa surface des sites fonctionnels, notamment hydroxyles, nécessitant l'utilisation d'un agent de couplage pour établir la liaison entre cette charge renforçante et l'élastomère diénique. À titre d'exemple, on peut citer des noirs de carbone partiellement ou intégralement recouverts de silice, ou des noirs de carbone modifiés par de la silice, tels que, à titre non limitatif, les charges de type « Ecoblack^{®} » de la série CRX2000 » ou de la série « CRX4000 » de la société Cabot Corporation.

L'homme du métier saura adapter le taux de charge renforçante selon l'utilisation concernée, notamment selon le type de pneumatiques concerné, par exemple pneumatique pour moto, pour véhicule de tourisme ou encore pour véhicule utilitaire tel que camionnette ou poids lourd. De préférence, ce taux de charge renforçante total (noir de carbone et/ou charge renforçante inorganique, de préférence noir de carbone et/ou silice) est compris dans un domaine allant de 10 à 200 pce, plus préférentiellement de 30 à 180 pce, et encore plus préférentiellement de 40 à 160 pce ; l'optimum étant de manière connue différent selon les applications particulières visées.

Selon une variante de l'invention, la charge renforçante est majoritairement autre que du noir de carbone, c'est-à-dire qu'elle comprend plus de 50 % en poids du poids total de la charge renforçante, d'une ou de plusieurs charges renforçantes autres que du noir de carbone, notamment une charge inorganique renforçante telle que la silice, voire elle est exclusivement constituée d'une telle charge (c'est-à-dire exclusivement constituée d'une charge inorganique renforçante, de préférence exclusivement constituée de silice). Selon cette variante, lorsque du noir de carbone est également présent, il peut être utilisé à un taux inférieur à 20 pce, plus préférentiellement inférieur à 10 pce (par exemple entre 0,5 et 20 pce, notamment de 1 à 10 pce).

Lorsque la charge renforçante comprend une charge inorganique renforçante nécessitant l'utilisation d'un agent de couplage pour établir la liaison entre la charge renforçante et l'élastomère diénique, la composition de caoutchouc selon l'invention comprend en outre, de manière classique, un agent susceptible d'assurer efficacement cette liaison. Lorsque la silice est présente dans la composition de caoutchouc selon l'invention à titre de charge renforçante, on peut utiliser comme agent de couplage des organosilanes. Préférentiellement, les organosilanes sont choisis dans le groupe constitué par les organosilanes polysulfurés (symétriques ou asymétriques) tels que le tétrasulfure de bis(3-triéthoxysilylpropyl), en abrégé TESPT commercialisé sous la dénomination « Si69 » par la société Evonik ou le disulfure de bis-(triéthoxysilylpropyle), en abrégé TESPD commercialisé sous la dénomination « Si75 » par la société Evonik, les polyorganosiloxanes, les mercaptosilanes, les mercaptosilanes bloqués, tels que l'octanethioate de S-(3-(triéthoxysilyl)propyle)commercialisé par la société Momentive sous la dénomination « NXT Silane ». Plus préférentiellement, l'organosilane est un organosilane polysulfuré.

Dans la composition selon l'invention, le taux d'agent de couplage est avantageusement inférieur à 20 pce, étant entendu qu'il est en générale souhaitable d'en utiliser le moins possible. Son taux est préférentiellement compris entre 0,5 pce et 12 pce. La présence de l'agent de couplage dépend de celle de la charge inorganique renforçante. Il est typiquement de l'ordre de 0,5 % à 15 % en poids par rapport à la quantité de charge inorganique renforçante.

### Système de réticulation chimique

La composition de caoutchouc selon l'invention est également à base d'au moins un système de réticulation chimique.

Le système de réticulation peut être tout type de système connu de l'homme de l'art dans le domaine des compositions de caoutchouc pour pneumatique. Il peut notamment être à base de soufre, et/ou de peroxyde et/ou de bismaléimides.

De manière préférentielle, le système de réticulation est à base de soufre, on parle alors d'un système de vulcanisation. Le soufre peut être apporté sous toute forme, notamment sous forme de soufre moléculaire, ou d'un agent donneur de soufre. Au moins un accélérateur de vulcanisation est également préférentiellement présent, et, de manière optionnelle, préférentielle également, on peut utiliser divers activateurs de vulcanisation connus tels qu'oxyde de zinc, acide stéarique ou composé équivalent tels que les sels d'acide stéarique et sels de métaux de transition, dérivés guanidiques (en particulier diphénylguanidine), ou encore des retardateurs de vulcanisation connus.

Le soufre est utilisé à un taux préférentiel compris entre 0,5 et 12 pce, en particulier entre 0,5 et 5 pce. L'accélérateur de vulcanisation est utilisé à un taux préférentiel compris entre 0,5 et 10 pce, plus préférentiellement compris entre 0,5 et 5,0 pce.

On peut utiliser comme accélérateur tout composé susceptible d'agir comme accélérateur de vulcanisation des élastomères diéniques en présence de soufre, notamment des accélérateurs du type thiazoles ainsi que leurs dérivés, des accélérateurs de types sulfénamides, thiurames, dithiocarbamates, dithiophosphates, thiourées et xanthates. A titre d'exemples de tels accélérateurs, on peut citer notamment les composés suivants : disulfure de 2-mercaptobenzothiazyle (en abrégé « MBTS »), N-cyclohexyl-2-benzothiazyle sulfénamide (« CBS »), N,N-dicyclohexyl-2-benzothiazyle sulfénamide (« DCBS »), N-ter-butyl-2-benzothiazyle sulfénamide (« TBBS »), N-ter-butyl-2-benzothiazyle sulfénimide (« TBSI »), disulfure de tétrabenzylthiurame (« TBZTD »), dibenzyldithiocarbamate de zinc (« ZBEC ») et les mélanges de ces composés.

A titre de composés peroxydes utilisables comme système de réticulation chimique, on peut citer les acyl peroxydes, par exemple le benzoyl peroxyde ou le p-chlorobenzoyl peroxyde, les cétones peroxydes, par exemple le méthyl éthyl cétone peroxyde, les peroxyesters, par exemple le t-butylperoxyacétate, le t-butylperoxybenzoate et le t-butylperoxyphtalate, les alkyl peroxides, par exemple le dicumyl peroxyde, le di-t-butyl peroxybenzoate et le 1,3-bis(t-butyl peroxyisopropyl)benzène, les hydroperoxydes, par exemple le t-butyl hydroperoxyde.

### Un plastifiant

Selon un mode de réalisation, la composition de caoutchouc peut comprendre en outre au moins un plastifiant.

De manière connue de l'homme de l'art des compositions de caoutchouc pour pneumatique, ce plastifiant est de préférence choisi parmi les résines hydrocarbonées de haute température de transition vitreuse (Tg), les résines hydrocarbonées de faible Tg, les huiles plastifiantes, et leurs mélanges. De préférence, le plastifiant est choisi parmi les résines hydrocarbonées de haute Tg, les huiles plastifiantes, et leurs mélanges.

Par définition, une résine hydrocarbonée de haute Tg est par définition un solide à température et pression ambiante (20°C, 1 atm), tandis qu'une huile plastifiante est liquide à température ambiante et qu'une résine hydrocarbonée de faible Tg est visqueuse à température ambiante.

Le taux total de plastifiants dans la composition est compris dans un domaine allant de 1 à 150 pce, de préférence dans un domaine allant de 2 à 120 pce.

Les résines hydrocarbonées, appelées aussi résines plastifiantes hydrocarbonées, sont des polymères bien connus de l'homme du métier, essentiellement à base de carbone et hydrogène mais pouvant comporter d'autres types d'atomes, par exemple l'oxygène, utilisables en particulier comme agents plastifiants ou agents tackifiants dans des matrices polymériques. Elles sont par nature au moins partiellement miscibles (i.e., compatibles) aux taux utilisés avec les compositions de polymères auxquelles elles sont destinées, de manière à agir comme de véritables agents diluants. Elles ont été décrites par exemple dans l'ouvrage intitulé « Hydrocarbon Resins » de R. Mildenberg, M. Zander et G. Collin (New York, VCH, 1997, ISBN 3-527-28617-9) dont le chapitre 5 est consacré à leurs applications, notamment en caoutchouterie pneumatique (5.5. « Rubber Tires and Mechanical Goods »). De manière connue, ces résines hydrocarbonées peuvent être qualifiées aussi de résines thermoplastiques en ce sens qu'elles se ramollissent par chauffage et peuvent ainsi être moulées.

Le point de ramollissement des résines hydrocarbonées est mesuré selon la norme ISO 4625 (méthode « Ring and Ball »). La Tg est mesurée selon la norme ASTM D3418 (2015). La macrostructure (Mw, Mn et Ip) de la résine hydrocarbonée est déterminée par chromatographie d'exclusion stérique (SEC) : solvant tétrahydrofurane ; température 35°C ; concentration 1 g/l ; débit 1 mL/min ; solution filtrée sur filtre de porosité 0,45 µm avant injection ; étalonnage de Moore avec des étalons de polystyrène ; jeu de 3 colonnes « WATERS » en série (« STYRAGEL »" HR4E, HR1 et HR0.5) ; détection par réfractomètre différentiel (« WATERS 2410») et son logiciel d'exploitation associé ("WATERS EMPOWER").

Les résines hydrocarbonées peuvent être aliphatiques, ou aromatiques ou encore du type aliphatique/ aromatique c'est-à-dire à base de monomères aliphatiques et/ou aromatiques. Elles peuvent être naturelles ou synthétiques, à base ou non de pétrole (si tel est le cas, connues aussi sous le nom de résines de pétrole).

A titre de monomères aromatiques conviennent par exemple le styrène, l'alpha-méthylstyrène, l'indène, l'ortho-, méta-, para-méthylstyrène, le vinyle-toluène, le para-tertiobutylstyrène, les méthoxystyrènes, les chlorostyrènes, le vinylmésitylène, le divinylbenzène, le vinylnaphtalène, tout monomère vinylaromatique issu d'une coupe C9 (ou plus généralement d'une coupe C8 à C10). De préférence, le monomère vinylaromatique est du styrène ou un monomère vinylaromatique issu d'une coupe C9 (ou plus généralement d'une coupe C8 à C10).

Selon un mode de réalisation particulièrement préférentiel, la résine plastifiante hydrocarbonée est choisie dans le groupe constitué par les résines d'homopolymères ou copolymères de cyclopentadiène (en abrégé CPD) ou dicyclopentadiène (en abrégé DCPD), les résines d'homopolymères ou copolymères terpène, les résines d'homopolymères ou copolymères terpène phénol, les résines d'homopolymères ou copolymères de coupe C5, les résines d'homopolymères ou copolymères de coupe C9, les résines d'homopolymères et copolymères d'alpha-méthyl-styrène et les mélanges de ces résines.

De manière connue, les résines hydrocarbonées de haute Tg sont des résines hydrocarbonées, thermoplastiques, dont la Tg est supérieure à 20°C.

De préférence, la résine plastifiante hydrocarbonée de haute Tg présente au moins une quelconque des caractéristiques suivantes :
- une Tg supérieure à 30°C ;
- une masse moléculaire moyenne en nombre (Mn) comprise entre 300 et 2000 g/mol, plus préférentiellement entre 400 et 1500 g/mol ;
- un indice de polymolécularité (Ip) inférieur à 3, plus préférentiellement inférieur à 2 (rappel : Ip = Mw/Mn avec Mw masse moléculaire moyenne en poids).

Plus préférentiellement, cette résine plastifiante hydrocarbonée de haute Tg présente l'ensemble des caractéristiques préférentielles ci-dessus.

Les résines hydrocarbonées de haute Tg préférentielles ci-dessus sont bien connues de l'homme du métier et disponibles commercialement, par exemple vendues en ce qui concerne les :
- résines polylimonène : par la société DRT sous la dénomination "Dercolyte L120" (Mn=625 g/mol ; Mw=1010 g/mol ; Ip=1,6 ; Tg=72°C) ou par la société ARIZONA sous la dénomination "Sylvagum TR7125C" (Mn=630 g/mol ; Mw=950 g/mol ; Ip=1,5 ; Tg=70°C) ;
- résines de copolymère coupe C5/ vinylaromatique, notamment coupe C5/ styrène ou coupe C5/ coupe C9 : par Neville Chemical Company sous les dénominations « Super Nevtac 78 », « Super Nevtac 85 » ou « Super Nevtac 99 », par Goodyear Chemicals sous dénomination « Wingtack Extra », par Kolon sous dénominations « Hikorez T1095 » et « Hikorez T1100 », par Exxon sous dénominations « Escorez 2101 » et « Escorez 1273 »;
- résines de copolymère limonène/ styrène : par DRT sous dénomination « Dercolyte TS 105 » de la société DRT, par ARIZONA Chemical Company sous dénominations « ZT115LT » et « ZT5100 ».

Le plastifiant peut contenir également une huile d'extension (ou huile plastifiante) liquide à 20 °C, dit à « basse Tg », c'est-à-dire qui par définition présente une Tg inférieure à -20 °C, de préférence inférieure à -40 °C.

Toute huile d'extension, qu'elle soit de nature aromatique ou non-aromatique connue pour ses propriétés plastifiantes vis-à-vis d'élastomères, est utilisable. A température ambiante (20°C), ces huiles, plus ou moins visqueuses, sont des liquides (c'est-à-dire, pour rappel, des substances ayant la capacité de prendre à terme la forme de leur contenant), par différence notamment avec les résines hydrocarbonées de haute Tg qui sont par nature solides à température ambiante.

Conviennent particulièrement les huiles plastifiantes choisies dans le groupe constitué par les huiles naphténiques (à basse ou haute viscosité, notamment hydrogénées ou non), les huiles paraffiniques, les huiles MES (Medium Extracted Solvates), les huiles TDAE (Treated Distillate Aromatic Extracts), les huiles RAE (Residual Aromatic Extract oils), les huiles TRAE (Treated Residual Aromatic Extract) et les huiles SRAE (Safety Residual Aromatic Extract oils), les huiles minérales, les huiles végétales, les plastifiants éthers, les plastifiants esters, les plastifiants sulfonates et les mélanges de ces composés.

### Additifs

Les compositions de caoutchouc conformes à l'invention peuvent comporter également tout ou partie des additifs et agents de mise en oeuvre usuels, connus de l'homme de l'art et habituellement utilisés dans les compositions de caoutchouc pour pneumatiques, en particulier de bandes de roulement, comme par exemple des charges non renforçantes, des pigments, des agents de protection tels que cires anti-ozone, anti-ozonants chimiques, anti-oxydants, des agents anti-fatigue, des résines renforçantes (telles que décrites par exemple dans la demande WO 02/10269).

### Procédé de fabrication des compositions de caoutchouc selon l'invention

La composition de caoutchouc conforme à l'invention est fabriquée dans des mélangeurs appropriés, en utilisant deux phases de préparation successives bien connues de l'homme du métier :une première phase de travail ou malaxage thermomécanique (phase dite « non-productive »), à haute température, jusqu'à une température maximale comprise dans un domaine allant de 130°C à 200°C, de préférence allant de 145°C à 185°C, suivie d'une seconde phase de travail mécanique (parfois qualifiée de phase « productive ») à plus basse température, typiquement inférieure à 120°C, par exemple entre 30°C et 100°C, phase de finition au cours de laquelle est incorporé le système de réticulation chimique.

De manière générale, tous les constituants de base de la composition de l'invention, à l'exception du système de réticulation chimique, à savoir le composé de formule (I) selon le mode de réalisation choisi, de préférence le composé de formule (Ib), plus préférentiellement le composé de formule (III), la ou les charges renforçantes, l'agent de couplage le cas échéant, sont incorporés de manière intime, par malaxage, à l'élastomère diénique ou aux élastomères diéniques au cours de la première phase dite non-productive, c'est-à-dire que l'on introduit dans le mélangeur approprié tel qu'un mélangeur interne usuel (par exemple de type « Banbury ») et que l'on malaxe thermomécaniquement, en une ou plusieurs étapes, au moins ces différents constituants de base jusqu'à atteindre la température maximale comprise dans un domaine allant de 130°C à 200°C, de préférence comprise dans un domaine allant de 145°C à 185°C. La durée totale du malaxage, dans cette phase non-productive, pendant une durée généralement comprise entre 2 et 10 minutes.

De manière générale, la seconde phase de travail mécanique (phase dite « productive »), qui est réalisée dans un mélangeur externe tel qu'un mélangeur à cylindres, après refroidissement du mélange obtenu au cours de la première phase non-productive jusqu'à une plus basse température, typiquement inférieure à 120°C, par exemple entre 30°C et 100°C.On incorpore alors le système de réticulation, et le tout est alors mélangé pendant quelques minutes, par exemple entre 5 et 15 min

La composition finale ainsi obtenue est ensuite calandrée par exemple sous la forme d'une feuille ou d'une plaque, notamment pour une caractérisation au laboratoire, ou encore extrudée sous la forme d'un semi-fini (ou profilé) de caoutchouc utilisable par exemple comme une bande de roulement de pneumatique.

La composition peut être soit à l'état cru (avant réticulation ou vulcanisation), soit à l'état cuit (après réticulation ou vulcanisation), peut être un produit semi-fini qui peut être utilisé dans un pneumatique.

La réticulation de la composition peut être conduite de manière connue de l'homme du métier, par exemple à une température comprise entre 130°C et 200°C, sous pression.

Selon le premier mode de réalisation de l'invention, l'élastomère diénique a été greffé par le composé de formule (I), de préférence le composé de formule (Ib), plus préférentiellement le composé de formule (III), préalablement à la fabrication de la composition de caoutchouc. Ainsi, dans ce cas, c'est l'élastomère diénique greffé qui est introduit au cours de la première phase dite non-productive. Ainsi selon ce premier mode de réalisation du procédé, celui-ci comprend les étapes suivantes:
- modifier l'élastomère diénique par greffage en solution ou en masse du composé de formule (I) telle que défini précédemment, de préférence le composé de formule (Ib), plus préférentiellement le composé de formule (III);
- incorporer à l'élastomère diénique ainsi greffé par le composé de formule (I)(ou les composés préférés), la charge renforçante et tous les constituants de base de la composition, à l'exception du système de réticulation chimique, en malaxant thermomécaniquement le tout, en une ou plusieurs fois, jusqu'à atteindre une température maximale comprise dans un domaine allant de 130°C à 200°C, de préférence de 145°C à 185°C ;
- refroidir le mélange de l'étape précédente à une température inférieure ou égale à 100°C ;
- incorporer ensuite le système de réticulation chimique ;
- malaxer le mélange de l'étape précédente jusqu'à une température maximale inférieure ou égale à 120°C ; puis optionnellement
- extruder ou calandrer la composition de caoutchouc ainsi obtenue.

Avantageusement, les étapes de ce premier mode de réalisation du procédé sont successives.

Le greffage de l'élastomère diénique se fait par réaction dudit élastomère diénique avec l'oxyde de nitrile du composé de formule (I), de préférence du composé de formule (Ib), plus préférentiellement du composé de formule (III). Lors de cette réaction, ce groupement oxyde de nitrile forme une liaison covalente avec la chaîne du polymère. Le greffage du composé de formule (I), de préférence du composé de formule (Ib), plus préférentiellement du composé de formule (III), est effectué par cycloaddition [3+2] de l'oxyde de nitrile et une insaturation de la chaîne du polymère initial. Un mécanisme de la cycloaddition [3+2] est illustré dans le document WO2012/007441.

Le greffage du composé de formule (I), de préférence du composé de formule (Ib), plus préférentiellement du composé de formule (III), sur l'élastomère diénique, peut être réalisé en masse, par exemple dans une extrudeuse, dans un mélangeur interne ou dans un mélangeur externe tel qu'un mélangeur à cylindres. Il peut aussi être réalisé en solution, en continu ou en discontinu. L'élastomère diénique ainsi modifié peut être séparé de sa solution par tout type de moyen connu par l'homme du métier et en particulier par une opération de stripping à la vapeur d'eau.

Selon l'invention, l'élastomère diénique porte le long de sa chaîne un ou plusieurs groupes pendants issus de la réaction de greffage du composé de formule (I), de préférence du composé de formule (Ib), plus préférentiellement du composé de formule (III), tels que définis ci-dessus. Avantageusement, ces groupes pendants sont répartis de façon aléatoire le long de la chaîne.

Selon un mode de réalisation préféré, le taux molaire de greffage du composé de formule (I), de préférence du composé de formule (Ib), plus préférentiellement du composé de formule (III), est compris dans un domaine allant de 0,01 % à 5 %, de préférence de 0,01 % à 1 %, plus préférentiellement de 0,1 à 1 %.

Par « taux molaire de greffage » on entend le nombre de mol de composé de formule (I), de préférence du composé de formule (Ib), plus préférentiellement du composé de formule (III), greffé sur l'élastomère diénique pour 100 moles d'unité monomère constituant l'élastomère diénique. Le taux molaire de greffage peut être déterminé par les méthodes conventionnelles d'analyses des élastomères diéniques, telles que par exemple l'analyse RMN ¹H.

Selon le deuxième mode de réalisation de l'invention, le greffage de l'élastomère diénique par le composé de formule (I), de préférence le composé de formule (Ib), plus préférentiellement encore le composé de formule (III) est effectué concomitamment à la fabrication de la composition de caoutchouc. Dans ce cas, tant l'élastomère diénique non encore greffé que le composé de formule (I) (ou les composés préférés) sont introduits au cours de la première phase dite non-productive. De manière préférentielle, la charge renforçante est alors ajoutée subséquemment au cours de cette même phase non-productive afin de prévenir toute réaction parasite avec le composé de formule (I).

Ainsi, selon ce deuxième mode de réalisation du procédé, celui-ci comprend les étapes suivantes:
- incorporer au cours du mélangeage en masse à l'élastomère diénique, le composé de formule (I) tel que défini précédemment, de préférence le composé de formule (Ib), plus préférentiellement le composé de formule (III), à une température notamment comprise dans un domaine allant de 60°C à 140°C et pendant une durée notamment comprise dans un domaine allant de 30 secondes à 3 minutes ; ces conditions de température et de temps permettant avantageusement d'obtenir un rendement de greffage supérieur ou égal à 60%, plus préférentiellement supérieur ou égal à 80%, et, de préférence subséquemment, la charge renforçante, ainsi que tous les constituants de base de la composition, à l'exception du système de réticulation chimique, en malaxant thermomécaniquement le tout, en une ou plusieurs fois, jusqu'à atteindre une température maximale comprise dans un domaine allant de 130°C à 200°C, de préférence de 145°C à 185°C ;
- refroidir l'ensemble à une température inférieure ou égale à 100°C ;
- incorporer ensuite le système de réticulation chimique ;
- malaxer le tout jusqu'à une température maximale inférieure ou égale à 120°C ; et optionnellement
- extruder ou calandrer la composition de caoutchouc ainsi obtenue.

Avantageusement, les étapes de ce deuxième mode de réalisation du procédé sont successives.

### Compositions de caoutchouc selon l'invention comprenant en outre un composé de formule (V)

Selon une variante de l'invention, la composition de caoutchouc comprend en outre au moins un composé de formule (V), éventuellement déjà greffé sur l'élastomère diénique, ledit élastomère diénique pouvant potentiellement déjà comprend au moins un composé de formule (I) tel que défini précédemment greffé sur sa chaîne 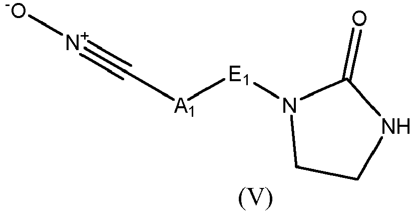 dans laquelle :
- A₁ représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
- E₁ représente un groupe divalent hydrocarboné comprenant éventuellement un ou plusieurs hétéroatomes.

De préférence, le groupement A₁ du composé de formule (V) est un cycle arènediyle en C₆-C₁₄ éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes. Plus préférentiellement, ce groupement A₁ est un cycle arènediyle en C₆-C₁₄, éventuellement substitué par une ou plusieurs chaînes hydrocarbonées, identiques ou différentes, saturées en C₁-C₂₄, linéaires ou ramifiées, éventuellement substituées ou interrompues par un ou plusieurs hétéroatomes d'azote, de soufre ou d'oxygène Plus préférentiellement encore, ce groupement A₁ est un cycle arènediyle en C₆-C₁₄, éventuellement substitué par un ou plusieurs substituants, identiques ou différents, choisis dans le groupe constitué par un alkyle en C₁-C₁₂, (plus préférentiellement en C₁-C₆, plus préférentiellement encore en C₁-C₄), un groupement-OR', un groupement -NHR' et un groupement -SR' où R' est un groupe alkyle en C₁-C₁₂, plus préférentiellement en C₁-C₆, plus préférentiellement encore en C₁-C₄.

De préférence, le composé de formule (V) est choisi parmi les composés de formule (Va) et (Vb) 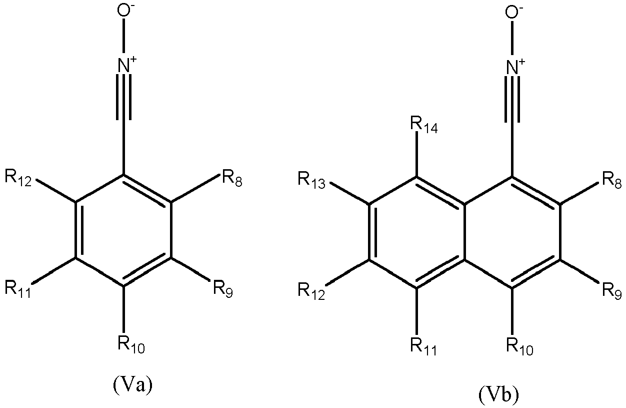 dans lesquelles :
- un groupement choisi parmi R₈ à R₁₂ de la formule (Va) et un groupement choisi parmi R₈ à R₁₄ de la formule (Vb) désignent le groupe de formule (VI) suivante 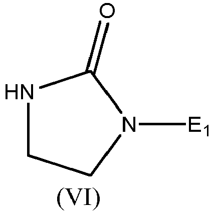 dans laquelle E₁ est tel que défini ci-dessus ; et
- les quatre groupements de la formule (Va) choisis parmi R₈ à R₁₂ autres que celui désignant le groupe de formule (VI) et les six groupements de la formule (Vb) choisis parmi R₈ à R₁₄ autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée éventuellement substituée ou interrompue par un ou plusieurs hétéroatomes.

Préférentiellement, dans les composés de formule (Va) et (Vb), les quatre groupements de la formule (Va) choisis parmi R₁ à R₅ autres que celui désignant le groupe de formule (VI) et les six groupements de la formule (Vb) choisis parmi R₁ à R₇ autres que celui désignant le groupe de formule (VI), identiques ou différents, représentent indépendamment les uns des autres, un atome d'hydrogène ou une chaîne hydrocarbonée aliphatique, saturée, linéaire ou ramifiée, en C₁-C₂₄, éventuellement substituée ou interrompue par un ou plusieurs hétéroatomes.

Plus préférentiellement encore, dans les composés de formule (Va) et (Vb), les quatre groupements de la formule (Va) choisis parmi R₁ à R₅ autres que celui désignant le groupe de formule (VI) et les six groupements de la formule (Vb) choisis parmi R₁ à R₇ autres que celui désignant le groupe de formule (VI), identiques ou différents, sont choisis dans le groupe formé par un atome d'hydrogène, un alkyle en C₁-C₁₂ (plus préférentiellement en C₁-C₆, plus préférentiellement encore en C₁-C₄), un groupement-OR', un groupement -NHR' et un groupement -SR' où R' est un alkyle en C₁-C₁₂, plus préférentiellement en C₁-C₆, plus préférentiellement encore en C₁-C₄.

Selon un mode de réalisation préférée de l'invention, dans la formule (Va), R₉ représente un groupe de formule (VI) tel que défini ci-dessus et R₈, R₁₀, R₁₁ et R₁₂, identiques ou différents, représentent un atome d'hydrogène ou une chaîne hydrocarbonée aliphatique, de préférence saturée, linéaire ou ramifiée, en C₁-C₂₄, éventuellement substituée ou interrompue par un ou plusieurs hétéroatomes. Plus préférentiellement, R₉ représente un groupe de formule (VI) tel que défini ci-dessus et R₈, R₁₀, R₁₁ et R₁₂, identiques ou différents sont choisis dans le groupe constitué par un atome d'hydrogène, un alkyle en C₁-C₁₂ (plus préférentiellement en C₁-C₆, plus préférentiellement encore en C1-C4), un groupement -OR', un groupement -NHR' et un groupement -SR' où R' est un alkyle en C₁-C₁₂, plus préférentiellement en C₁-C₆, plus préférentiellement encore en C₁-C₄.

Plus préférentiellement encore dans ce mode de réalisation, dans la formule (Va), R₉ représente un groupe de formule (VI) tel que défini ci-dessus, R₁₁ représente un atome d'hydrogène et R₈, R₁₀ et R₁₂ représentent une chaîne hydrocarbonée aliphatique, de préférence saturée, linéaire ou ramifiée en C₁-C₂₄, éventuellement substituée ou interrompue par un ou plusieurs hétéroatomes. Plus préférentiellement encore, R₉ représente un groupe de formule (VI) tel que défini ci-dessus, R₁₁ représente un atome d'hydrogène et R₈, R₁₀ et R₁₂, identiques ou différents, sont choisis dans le groupe constitué par un alkyle en C₁-C₁₂ (plus préférentiellement en C₁-C₆, plus préférentiellement encore en C₁-C₄), un groupement -OR', un groupement-NHR' et un groupement -SR' où R' est un alkyle en C₁-C₁₂, plus préférentiellement en C₁-C₆, plus préférentiellement encore en C₁-C₄.

Selon un autre mode de réalisation préférée de l'invention, dans la formule (Vb), R₈ représente un groupe de formule (VI) tel que défini ci-dessus et R₉ à R₁₄ identiques ou différents, représentent un atome d'hydrogène ou une chaîne hydrocarbonée aliphatique, de préférence saturée, linéaire ou ramifiée, en C₁-C₂₄, éventuellement substituée ou interrompue par un ou plusieurs hétéroatomes. Plus préférentiellement, R₈ représente un groupe de formule (VI) tel que défini ci-dessus et R₉ à R₁₄, identiques ou différents, sont choisis dans le groupe constitué par un atome d'hydrogène, un alkyle en C₁-C₁₂ (plus préférentiellement en C₁-C₆, plus préférentiellement encore en C₁-C₄), un groupement -OR', un groupement -NHR', un groupement -SR' où R' est un alkyle en C₁-C₁₂, plus préférentiellement en C₁-C₆, plus préférentiellement encore en C₁-C₄. Plus préférentiellement encore dans ce mode de réalisation, R₈ représente un groupe de formule (VI) tel que défini ci-dessus et R₉ à R₁₄, identiques, représentent un atome d'hydrogène.

Dans les composés de formule (V), (Va) et (Vb), le groupement E₁ est un groupe divalent hydrocarboné pouvant éventuellement contenir un ou plusieurs hétéroatome(s). Par « groupe divalent hydrocarboné » on entend au sens de la présente invention, un groupe espaceur (ou un groupe de liaison) formant un pont entre le groupement A₁ et le groupe imidazolidinone , ce groupe espaceur étant une chaîne hydrocarbonée, saturée ou insaturée, de préférence saturée, en C₁-C₂₄, linéaire ou ramifiée, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec l'oxyde de nitrile et le groupe imidazolidinone tel que défini ci-dessus.

Préférentiellement, dans les composés de formule (V), (Va) et (Vb), le groupement E₁ est une chaîne hydrocarbonée, linéaire ou ramifiée, de préférence saturée, en C₁-C₂₄, plus préférentiellement en C₁-C₁₀, encore plus préférentiellement en C₁-C₆, éventuellement interrompue par un ou plusieurs atomes d'azote, de soufre ou d'oxygène.

De préférence, dans les composés de formule (V), (Va) et (Vb), le groupement E₁ est choisi dans le groupe constitué par -R-, -NH-R-, -O-R- et -S-R- où R est un alkylène, linéaire ou ramifié, en C₁-C₂₄, de préférence en C₁-C₁₀, plus préférentiellement en C₁-C₆.

Plus préférentiellement encore, dans les composés de formule (V), (Va) et (Vb), le groupement E₁ est choisi dans le groupe constitué par -R- et -OR- où R est un alkylène, linéaire ou ramifié, en C₁-C₂₄, de préférence en C₁-C₁₀, plus préférentiellement en C₁-C₆.

Plus préférentiellement encore, dans les composés de formule (V), (Va) et (Vb), le groupement E₁ est choisi parmi -CH₂-, -CH₂-CH₂-, -CH₂-CH₂-CH2-, -CH₂-CH₂-CH₂-CH₂-, -O-CH₂-, -O-CH₂-CH₂-, -O-CH₂-CH₂-CH₂- et -O-CH₂-CH₂-CH₂-CH₂-.

Selon un mode de réalisation préférée, le composé de formule (V) est le composé de formule (Vb).

Dans ce mode de réalisation préférée, on préfère en particulier le composé de formule (Vb) tel que défini ci-dessus avec le groupement E₁ choisi dans le groupe constitué par -R- et -OR- où R est un alkylène, linéaire ou ramifié, en C₁-C₂₄, de préférence en C₁-C₁₀, plus préférentiellement en C₁-C₆.

Selon ce mode de réalisation, plus préférentiellement encore, le composé de formule (V) est le composé de formule (Vb) avec :
- R₈ représente un groupe de formule (VI) dans lequel le groupement E₁ est choisi dans le groupe constitué par -R- et -OR- où R est un alkylène, linéaire ou ramifié, en C₁-C₂₄, de préférence en C₁-C₁₀, plus préférentiellement en C₁-C₆ ; et
- R₉ à R₁₄ identiques ou différents, représentent un atome d'hydrogène ou une chaîne hydrocarbonée aliphatique, de préférence saturée, linéaire ou ramifiée, en C₁-C₂₄, éventuellement substituée ou interrompue par un ou plusieurs hétéroatomes.

Selon ce mode de réalisation, plus préférentiellement encore, le composé de formule (V) est le composé de formule (Vb) avec :
- R₈ représente un groupe de formule (VI) dans lequel le groupement E1 est choisi dans le groupe constitué par -R- et -OR- où R un alkylène, linéaire ou ramifié, en C₁-C₂₄, de préférence en C₁-C₁₀, plus préférentiellement en C₁-C₆ ; et
- R₉ à R₁₄, identiques ou différents, sont choisis dans le groupe formé par un atome d'hydrogène, un alkyle en C₁-C₁₂ (plus préférentiellement en C₁-C₆, plus préférentiellement encore en C₁-C₄), un groupement -OR', un groupement -NHR', un groupement -SR' où R' est un alkyle en C₁-C₁₂, plus préférentiellement en C₁-C₆, plus préférentiellement encore en C₁-C₄.

Selon ce mode de réalisation, plus préférentiellement encore, le composé de formule (V) est le composé de formule (Vb) avec :
- R₈ représente un groupe de formule (VI) dans lequel le groupement E₁ choisi dans le groupe constitué par -R- et -OR- où R un alkylène, linéaire ou ramifié, en C₁-C₂₄, de préférence en C₁-C₁₀, plus préférentiellement en C₁-C₆; et
- R₉ à R₁₄, identiques, représentent un atome d'hydrogène.

Selon un mode de réalisation préféré, le composé de formule (V) est choisi dans le groupe constitué par le composé de formule (VII) et de formule (VIII), plus préférentiellement encore par le composé de formule (VII)

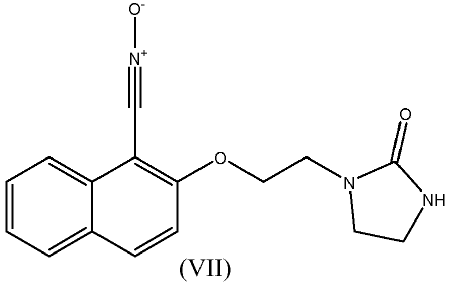

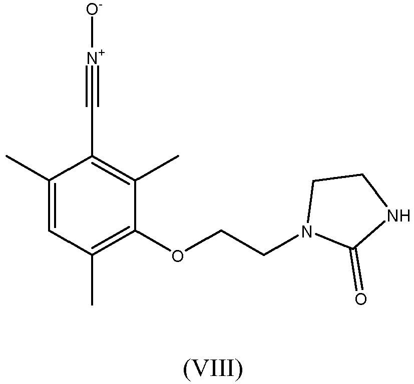

Le composé de formule V, en particulier, les composés de formule (Vb) et (VII), peuvent être, par exemple, synthétisés par le procédé décrit dans le document WO2019007881A1.

Préférentiellement, le taux molaire de composé de formule (V), de préférence de composé de formule (Vb), plus préférentiellement de composé (VII) dans la composition de caoutchouc selon l'invention, est compris dans un domaine allant de 0,01 à 5 % molaire, de préférence de 0,01 à 1 % molaire, plus préférentiellement de 0,1 à 1 % molaire, qu'il s'agisse indifféremment de motifs diéniques ou non diéniques.

Selon un mode de réalisation préféré, la composition de caoutchouc selon l'invention comprend au moins un composé de formule (Ib) tel que défini ci-dessus et au moins un composé de formule (Vb) tel que défini ci-dessus, lesdits composés étant éventuellement déjà greffés sur l'élastomère diénique.

De préférence, dans cette ce mode de réalisation, la composition de caoutchouc selon l'invention comprend au moins:
- un composé de formule (Ib) avec R₁ représente un groupe de formule (II) dans lequel le groupement E est choisi dans le groupe constitué par -R- et -OR- où R est un alkylène, linéaire ou ramifié, en C₁-C₂₄, de préférence en C₁-C₁₀, plus préférentiellement en C₁-C₆, groupement X qui est un atome de chlore ; et R₂ à R₇, identiques ou différents, sont choisis dans le groupe constitué par un atome d'hydrogène, un alkyle en C₁-C₁₂ (plus préférentiellement en C₁-C₆, plus préférentiellement encore en C₁-C₄), un groupement -OR', un groupement -NHR' et un groupement -SR' où R' est un alkyle en C₁-C₁₂, plus préférentiellement en C₁-C₆, plus préférentiellement encore en C₁-C₄ ; et
- un composé de formule (Vb) avec R₈ représente un groupe de formule (VI) dans lequel le groupement E₁ est choisi dans le groupe constitué par -R- et -OR- où R est un alkylène, linéaire ou ramifié, en C₁-C₂₄, de préférence en C₁-C₁₀, plus préférentiellement en C₁-C₆; et R₉ à R₁₄, identiques ou différents, sont choisis dans le groupe formé par un atome d'hydrogène, un alkyle en C₁-C₁₂ (plus préférentiellement en C₁-C₆, plus préférentiellement encore en C₁-C₄), un groupement -OR', un groupement -NHR', un groupement -SR' où R' est un alkyle en C₁-C₁₂, plus préférentiellement en C₁-C₆, plus préférentiellement encore en C₁-C₄ ; et
- lesdits composés étant éventuellement déjà greffés sur l'élastomère diénique.

Plus préférentiellement encore, dans ce mode de réalisation, la composition de caoutchouc selon l'invention comprend au moins un composé de formule (III) et au moins un composé de formule (VII), éventuellement déjà greffés sur l'élastomère diénique.

De manière surprenante, lorsque la composition de caoutchouc de l'invention comprend au moins un composé de formule (I) et au moins un composé de formule (V), de préférence au moins un composé de formule (Ib) et au moins un composé de formule (Vb), plus préférentiellement encore un composé de formule (III) et au moins un composé de formule (VII), éventuellement déjà greffés sur l'élastomère diénique, elle présente l'avantage d'avoir un compromis rigidité à cru/ hystérèse amélioré par rapport aux compositions de caoutchouc de l'art antérieur.

Préparation des compositions de caoutchouc de l'invention comprenant en outre le composé de formule (V)

Selon un premier mode de réalisation de ces compositions, l'élastomère diénique a été greffé par le composé de formule (1) et par le composé de formule (V), de préférence le composé de formule (Ib) et le composé de formule (Vb), plus préférentiellement le composé de formule (III) et le composé de formule (VII), préalablement à la fabrication de la composition de caoutchouc. Ainsi, dans ce cas, c'est l'élastomère diénique greffé avec les composés de formule (I) et (V) qui est introduit au cours de la première phase dite non-productive. Ainsi selon ce premier mode de réalisation du procédé, celui-ci comprend les étapes suivantes :
- modifier l'élastomère diénique par greffage en solution ou en masse des composés de formule (I) et de formule (V) tels que définis précédemment, de préférence les composés de formule (Ib) et de formule (Vb), plus préférentiellement le composé de formule (III) et le composé de formule (VII),
- incorporer à l'élastomère diénique ainsi greffé par les composés de formule (I) et de formule (V) (ou les composés préférés), la charge renforçante et tous les constituants de base de la composition, à l'exception du système de réticulation chimique, en malaxant thermomécaniquement le tout, en une ou plusieurs fois, jusqu'à atteindre une température maximale comprise dans un domaine allant de 130°C à 200°C, de préférence de 145°C à 185°C ;
- refroidir le mélange de l'étape précédente à une température inférieure ou égale à 100°C ;
- incorporer ensuite le système de réticulation chimique ;
- malaxer le mélange de l'étape précédente jusqu'à une température maximale inférieure ou égale à 120°C ; puis optionnellement
- extruder ou calandrer la composition de caoutchouc ainsi obtenue.

Avantageusement, les étapes de ce premier mode de réalisation du procédé sont successives.

Le greffage de l'élastomère diénique se fait par réaction dudit élastomère diénique avec l'oxyde de nitrile du composé de formule (I) et avec l'oxyde de nitrile du composé de formule (V). Comme expliqué précédemment, le greffage du composé de formule (I) et de celui du composé de formule (V) (ou des composés préférés précités) est effectué par cycloaddition [3+2] des oxydes de nitrile et des insaturations de la chaîne de l'élastomère diénique initiale.

Le greffage des composés de formule (I) et (V), de préférence des composés de formule (Ib) et (Vb), plus préférentiellement des composés de formule (III) et (VII), sur le polymère initial, peut être réalisé en masse, par exemple dans une extrudeuse, dans un mélangeur interne ou dans un mélangeur externe tel qu'un mélangeur à cylindres. Il peut aussi être réalisé en solution, en continu ou en discontinu. L'élastomère diénique ainsi modifié peut être séparé de sa solution par tout type de moyen connu par l'homme du métier et en particulier par une opération de stripping à la vapeur d'eau. Les composés de formule (I) et (V), de préférence des composés de formule (Ib) et (Vb), plus préférentiellement des composés de formule (III) et (VII), peuvent être introduits simultanément dans le mélangeur interne ou externe, ou simultanément dans le réacteur si le greffage s'effectue en solution. Ils peuvent aussi être introduits de manière décalée l'un par rapport à l'autre, l'ordre d'ayant pas d'importance pour le greffage de ces composés sur la chaîne de l'élastomère diénique initial.

Après greffage, l'élastomère diénique porte le long de sa chaîne un ou plusieurs groupes pendants issus de la réaction de greffage des composés de formule (I) et de formule (V) (ou des composés préférés précités). Avantageusement, ces groupes pendants sont répartis de façon aléatoire le long de la chaîne de l'élastomère diénique.

Le taux molaire de greffage du composé de formule (I), de préférence du composé de formule (Ib), plus préférentiellement du composé de formule (III), est compris dans un domaine allant de 0,01 % à 5 %, de préférence de 0,01 % à 1 %, plus préférentiellement de 0,1 à 1 % et le taux molaire de greffage du composé de formule (V), de préférence du composé de formule (Vb), plus préférentiellement du composé de formule (VII), est compris dans un domaine allant de 0,01 % à 5 %, de préférence de 0,01 % à 1 %, plus préférentiellement de 0,1 à 1 %.

Selon le deuxième mode de réalisation de ces compositions, le greffage de l'élastomère diénique par le composé de formule (I) et de formule (V), de préférence le composé de formule (Ib) et de formule (Vb), plus préférentiellement encore le composé de formule (III) et de formule (VII) est effectué concomitamment à la fabrication de la composition de caoutchouc. Dans ce cas, tant l'élastomère diénique non encore greffé que le composé de formule (I) et que le composé de formule (V) (ou les composés préférés) sont introduits au cours de la première phase dite non-productive. De manière préférentielle, la charge renforçante est alors ajoutée subséquemment au cours de cette même phase non-productive afin de prévenir toute réaction parasite avec le composé de formule (I) et le composé de formule (V).

Ainsi, selon ce deuxième mode de réalisation du procédé, celui-ci comprend les étapes suivantes :
- incorporer au cours du mélangeage en masse à l'élastomère diénique, le composé de formule (I) tel que défini précédemment, de préférence le composé de formule (Ib), plus préférentiellement le composé de formule (III), le composé de formule (V) tel que défini précédemment, de préférence le composé de formule (Vb), plus préférentiellement le composé de formule (VII) , à une température notamment comprise dans un domaine allant de 60°C à 140°C et pendant une durée notamment comprise dans un domaine allant de 30 secondes à 3 minutes ; ces conditions de température et de temps permettant avantageusement d'obtenir un rendement de greffage de préférence supérieur ou égal à 60%, plus préférentiellement supérieur ou égal à 80%, et, de préférence subséquemment, la charge renforçante, ainsi que tous les constituants de base de la composition, à l'exception du système de réticulation chimique, en malaxant thermomécaniquement le tout, en une ou plusieurs fois, jusqu'à atteindre une température maximale comprise dans un domaine allant de 130°C à 200°C, de préférence de 145°C à 185°C ;
- refroidir l'ensemble à une température inférieure ou égale à 100°C ;
- incorporer ensuite le système de réticulation chimique ;
- malaxer le tout jusqu'à une température maximale inférieure ou égale à 120°C ; et optionnellement
- extruder ou calandrer la composition de caoutchouc ainsi obtenue.

Avantageusement, les étapes de ce deuxième mode de réalisation du procédé sont successives.

Produits semi-finis pour pneumatiques et pneumatiques.

L'invention a également pour objet un article semi-fini en caoutchouc pour pneumatique comprenant une composition de caoutchouc selon l'invention telle que définie précédemment.

Un produit semi-fini est un produit en caoutchouc destiné à la fabrication de pneumatique. Ce peut être des bandes de roulement, des sous-couches, des nappes d'armature de sommet (par exemple des nappes de travail, des nappes de protection ou des nappes de frettage), de nappes d'armature de carcasse, des nappes de flancs, des nappes de bourrelets, des nappes de protecteurs, des nappes de sous-couches, des nappes de blocs de caoutchouc et autres nappes assurant l'interface entre les zones des pneumatiques. De préférence, l'article semi-fini est une bande de roulement.

Un autre objet de l'invention est un pneumatique comprenant au moins une composition de caoutchouc selon l'invention ou comprenant un article semi-fini pour pneumatique tel que décrit ci-dessus.

Le pneumatique selon l'invention peut être destiné à équiper notamment les véhicules sans moteur tels que les bicyclettes, les véhicules à moteur de type tourisme, les SUV (Sport Utility Vehicles), les deux roues (notamment les motos), les avions, les véhicules industriels choisis parmi les camionnettes, les poids-lourds (c'est-à-dire le métro, les bus, les engin routiers (camions et remorques)), les véhicules hors la route, tels que les engins agricoles ou de génie civil, les autres véhicules de transports ou de manutention.

En plus, des objets décrits précédemment, l'invention concerne au moins l'un des objets décrits aux réalisations suivantes :
1. Composition de caoutchouc à base d'au moins un élastomère diénique, d'au moins une charge renforçante, d'au moins un système de réticulation chimique et d'au moins un composé de formule (I) éventuellement déjà greffé sur ledit élastomère diénique dans laquelle : 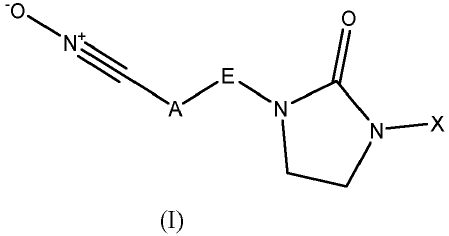
   - A représente un cycle arènediyle, éventuellement substitué par une ou plusieurs chaînes hydrocarbonées, identiques ou différentes, aliphatiques, de préférence saturées, linéaires ou ramifiés, éventuellement substituées ou interrompues par un ou plusieurs hétéroatomes ;
   - E représente un groupe divalent hydrocarboné comprenant éventuellement un ou plusieurs hétéroatomes ; et
   - X désigne un atome d'halogène, de préférence un atome de chlore.
2. Composition de caoutchouc selon la réalisation 1, dans laquelle l'élastomère diénique est choisi dans le groupe constitué par les copolymères d'éthylène-propylène-monomère diène, le caoutchouc butyle, le caoutchouc naturel, les polyisoprènes de synthèse, les polybutadiènes, les copolymères de butadiène, les copolymères d'isoprène et les mélanges de ces élastomères.
3. Composition de caoutchouc selon l'une quelconque des réalisations précédentes, dans laquelle le taux molaire de greffage du composé de formule (I) est compris dans un domaine allant de 0,01 % à 5 %, de préférence de 0,01 % à 1 %, plus préférentiellement de 0,1 à 1 %.
4. Composition de caoutchouc selon l'une quelconque des réalisations précédentes, dans laquelle le groupement A est un cycle arènediyle en C₆-C₁₄ éventuellement substitué par une ou plusieurs chaînes hydrocarbonées, identiques ou différentes, aliphatiques, de préférence, linéaires ou ramifiées, éventuellement substituées ou interrompues par un ou plusieurs hétéroatomes.
5. Composition de caoutchouc selon l'une quelconque des réalisations précédentes, dans laquelle le composé de formule (I) est choisi parmi les composés de formule (la) et (Ib) 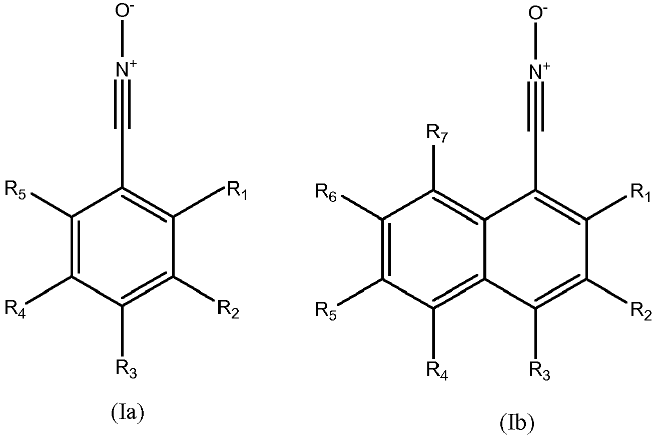 dans lesquelles :
   - un groupement choisi parmi R₁ à R₅ de la formule (Ia) et un groupement choisi parmi R₁ à R₇ de la formule (Ib) désignent le groupe de formule (II) suivante 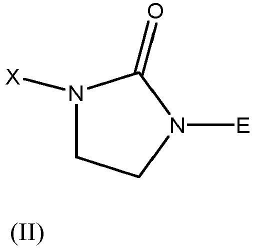 dans laquelle E et X sont tels que défini au point 1,
   - les quatre groupements de la formule (Ia) choisis parmi R₁ à R₅ autres que celui désignant le groupe de formule (II) et les six groupements de la formule (Ib) choisis parmi R₁ à R₇ autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.
6. Composition de caoutchouc selon l'une quelconque des réalisations précédentes, dans laquelle groupement E est une chaîne hydrocarbonée, linéaire ou ramifiée, de préférence saturée, en C₁-C₂₄, de préférence en C₁-C₁₀, plus préférentiellement en C₁-C₆, éventuellement interrompue par un ou plusieurs atomes d'azote, de soufre ou d'oxygène.
7. Composition de caoutchouc selon l'une quelconque des réalisations précédentes, dans laquelle le groupement E est choisi dans le groupe constitué par -R- et -OR- où R est un alkylène, linéaire ou ramifié, en C₁-C₂₄, de préférence en C₁-C₁₀, plus préférentiellement en C₁-C₆.
8. Composition de caoutchouc selon l'une quelconque des réalisations précédentes, dans laquelle le composé de formule (I) est le composé de formule (Ib).
9. Composition de caoutchouc selon l'une quelconque des réalisations précédentes, dans laquelle le composé de formule (I) est le composé de formule (III) 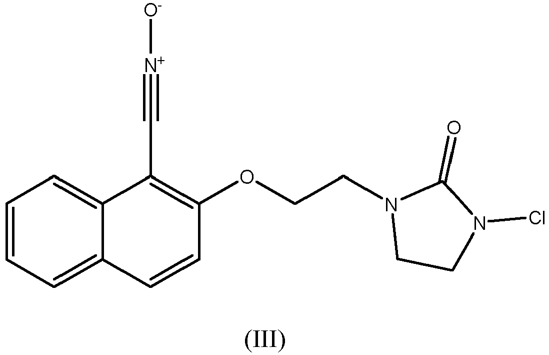
10. Composition de caoutchouc selon l'une quelconque des réalisations précédentes, comprenant en outre au moins un composé de formule (V), éventuellement déjà greffé sur l'élastomère diénique 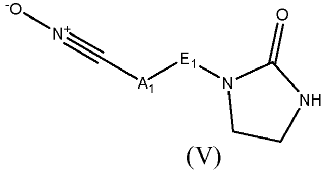 dans laquelle :
   - A₁ représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
   - E₁ représente un groupe divalent hydrocarboné comprenant éventuellement un ou plusieurs hétéroatomes.
11. Composition de caoutchouc selon la réalisation 10, dans laquelle le taux molaire de greffage du composé de formule (V) est compris dans un domaine allant de 0,01 % à 5 %, de préférence de 0,01 % à 1 %, plus préférentiellement de 0,1 à 1 %.
12. Composition de caoutchouc selon la réalisation 10 ou 11, dans laquelle le groupement A1 est un cycle arènediyle en C₆-C₁₄ éventuellement substitué par une ou plusieurs chaînes hydrocarbonées, identiques ou différentes, aliphatiques, de préférences, linéaires ou ramifiées, éventuellement substituées ou interrompues par un ou plusieurs hétéroatomes.
13. Composition de caoutchouc selon l'une quelconque des réalisations 10 à 12, dans laquelle le composé de formule (V) est choisi parmi les composés de formule (Va) et (Vb) 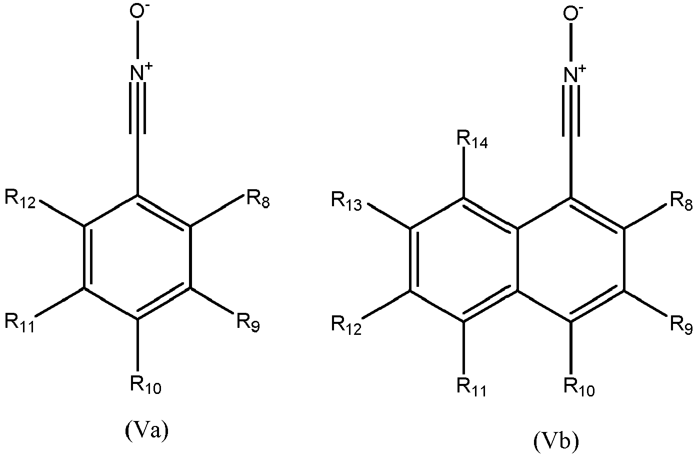 dans lesquelles :
   - un groupement choisi parmi R₈ à R₁₂ de la formule (Va) et un groupement choisi parmi R₈ à R₁₄ de la formule (Vb) désignent le groupe de formule (VI) suivante 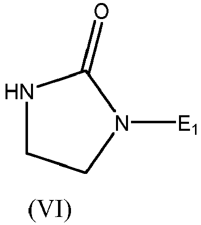 avec E₁ tel que défini à la réalisation 10 ; et
   - les quatre groupements de la formule (Va) choisis parmi R₈ à R₁₂ autres que celui désignant le groupe de formule (VI) et les six groupements de la formule (Vb) choisis parmi R₈ à R₁₄ autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.
14. Composition de caoutchouc selon l'une quelconque des réalisations 10 à 13, dans laquelle le groupement E₁ du composé de formule (V) est une chaîne hydrocarbonée, linéaire ou ramifiée, de préférence saturée, en C₁-C₂₄, de préférence en C₁-C₁₀, plus préférentiellement en C₁-C₆, éventuellement interrompue par un ou plusieurs atomes d'azote, de soufre ou d'oxygène.
15. Composition de caoutchouc selon l'une quelconque des réalisations 10 à 14, dans laquelle le groupement E₁ du composé de formule (V) est choisi dans le groupe constitué par -R- et -OR- où R est un alkylène, linéaire ou ramifié, en C₁-C₂₄, de préférence en C₁-C₁₀, plus préférentiellement en C₁-C₆.
16. Composition de caoutchouc selon l'une quelconque des réalisations 10 à 15, dans lequel le composé de formule (V) est le composé de formule (Vb).
17. Composition de caoutchouc selon l'une quelconque des réalisations 10 à 17, dans lequel le composé de formule (V) est le composé de formule (VII) 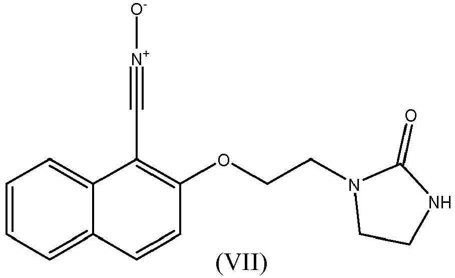
18. Composition de caoutchouc selon l'une quelconque des réalisations précédentes, dans laquelle la charge renforçante est un noir de carbone ou un mélange de noir de carbone.
19. Composition de caoutchouc selon l'une quelconque des réalisations précédentes, dans laquelle la charge renforçante est une charge inorganique renforçante ou un mélange de charge inorganique renforçante, de préférence une silice ou un mélange de silice.
20. Composition de caoutchouc selon l'une quelconque des réalisations précédentes, dans laquelle la charge renforçante est un mélange d'au moins un noir de carbone et d'au moins une charge inorganique renforçante, de préférence d'au moins un noir de carbone et d'au moins une silice.
21. Composition de caoutchouc selon la réalisation 19 ou 20, dans laquelle la composition comprend en outre un agent de couplage de la charge inorganique renforçante avec l'élastomère diénique.
22. Composition de caoutchouc selon la réalisation 21, dans laquelle ledit agent de couplage est choisi parmi le groupe constitué par les organosilanes polysulfurés, les polyorganosiloxanes, les mercaptosilanes et les mercaptosilanes bloqués.
23. Composition de caoutchouc selon l'une quelconque des réalisations précédentes, dans laquelle le taux de charge renforçante est compris dans un domaine allant de 10 à 200 pce, de préférence de 30 à 180 pce, plus préférentiellement de 40 à 160 pce.
24. Composition de caoutchouc selon l'une quelconque des réalisations précédentes, comprenant en outre un plastifiant.
25. Composition de caoutchouc selon la réalisation 22, dans laquelle le plastifiant est choisi dans le groupe constitué par les résines plastifiantes hydrocarbonées ayant une Tg supérieure à 20°C, les huiles plastifiantes, et leurs mélanges.
26. Article semi -fini en caoutchouc pour pneumatique, caractérisé en ce qu'il comprend une composition de caoutchouc telle que définie selon l'une quelconque des réalisations 1 à 25.
27. Pneumatique, caractérisé en ce qu'il comporte au moins un article semi-fini selon la réalisation 26 ou au moins une composition selon l'une quelconque des réalisations 1 à 25.

### EXEMPLES :

Les exemples qui suivent permettent d'illustrer l'invention, cette dernière ne saurait cependant être limitée à ces seuls exemples.

### 1 Tests et mesures utilisés

### 1.1 Détermination de la température de transition vitreuse

La température de transition vitreuse Tg des polymères sont mesures au moyen d'un calorimètre différentiel (« Differential Scanning Calorimeter ») selon la norme ASTM D3418 (2015).

### 1.2 Mesure des masses molaires moyennes en nombre (Mn), en poids (Mw) et de l'indice de polydispersité pour les élastomères

On utilise la chromatographie d'exclusion stérique ou SEC (Size Exclusion Chromatography). La SEC permet de séparer les macromolécules en solution suivant leur taille à travers des colonnes remplies d'un gel poreux. Les macromolécules sont séparées suivant leur volume hydrodynamique, les plus volumineuses étant éluées en premier.

Sans être une méthode absolue, la SEC permet d'appréhender la distribution des masses molaires d'un élastomère. À partir de produits étalons commerciaux, les différentes masses molaires moyennes en nombre (Mn) et en poids (Mw) peuvent être déterminées et l'indice de polymolécularité (Ip = Mw/Mn) calculé via un étalonnage dit de MOORE.

Préparation de l'échantillon d'élastomère à tester:
Il n'y a pas de traitement particulier de l'échantillon de l'élastomère avant analyse. Celui-ci est simplement solubilisé à une concentration d'environ 1 g/L, dans du chloroforme ou dans le mélange suivant : tétrahydrofurane + 1 % vol. de diisopropylamine + 1 % vol. de triéthylamine + 1 % vol. d'eau distillée (% vol. = % volume). Puis, la solution est filtrée sur filtre de porosité 0,45 µm avant injection.

### Analyse SEC

L'appareillage utilisé est un chromatographe « WATERS alliance ». Le solvant d'élution est le mélange suivant : tétrahydrofurane + 1 % vol. de diisopropylamine + 1 % vol. de triéthylamine ou du chloroforme selon le solvant utilisé pour la mise en solution de l'élastomère. Le débit est de 0,7 mL/min, la température du système de 35°C et la durée d'analyse de 90 min. On utilise un jeu de quatre colonnes WATERS en série, de dénominations commerciales «STYRAGEL HMW7», «STYRAGEL HMW6E» et deux «STYRAGEL HT6E».

Le volume injecté de la solution de l'échantillon de l'élastomère est 100 µL. Le détecteur est un réfractomètre différentiel « WATERS 2410 » de longueur d'onde 810 nm. Le logiciel d'exploitation des données chromatographiques est le système «WATERS EM POWER». Les masses molaires moyennes calculées sont relatives à une courbe d'étalonnage réalisée à partir de polystyrènes étalons commerciaux « PSS READY CAL-KIT ».

### 1.3 Caractérisations des molécules

L'analyse structurale ainsi que la détermination des puretés molaires des molécules de synthèse sont réalisées par une analyse RMN. Les spectres sont acquis sur un spectromètre « Avance 3 400 MHz BRUKER » équipé d'une sonde «large bande BBFO-zgrad 5 mm». L'expérience RMN ¹H quantitative utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de « lock ». Par exemple, la calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf tableaux d'attributions). Les quantifications molaires sont réalisées à partir du spectre RMN 1D ¹H quantitatif.

L'analyse par spectrométrie de masse est réalisée en injection directe par un mode d'ionisation en électrospray (ID/ESI). Les analyses ont été réalisées sur un spectromètre HCT Bruker (débit 600 µl/min, pression du gaz nébuliseur 10 psi, débit du gaz nébuliseur 4 1/min).

### 1.4 Caractérisations des composés greffés sur les élastomères diéniques

La détermination du taux molaire des composés greffés sur les élastomères diéniques est réalisée par une analyse RMN. Les spectres sont acquis sur un spectromètre « 500 MHz BRUKER » équipé d'une «CryoSonde BBFO-zgrad-5 mm». L'expérience RMN ¹H quantitative, utilise une séquence simple impulsion 30° et un délai de répétition de 5 secondes entre chaque acquisition. Les échantillons sont solubilisés dans le chloroforme deutéré (CDCl₃) dans le but d'obtenir un signal de «lock». Des expériences RMN 2D ont permis de vérifier la nature du motif greffé grâce aux déplacements chimiques des atomes de carbone et de proton.

### 1.5 Propriétés dynamiques

Les propriétés dynamiques G* et tan(δ)max sont mesurées sur un viscoanalyseur (Metravib VA4000), selon la norme ASTM D5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10 Hz, dans les conditions normales de température (23°C) selon la norme ASTM D1349-09. On effectue un balayage en amplitude de déformation de 0,1 % à 50 % crête-crête (cycle aller) puis de 50 % à 0,1 % crête-crête (cycle retour).

Les résultats exploités sont le module complexe de cisaillement dynamique G*à 23°C et le facteur de perte dynamique tan(δ) à 23°C. Pour l'aller, on enregistre la valeur du module complexe de cisaillement dynamique G* à 50 % de déformation, noter G^{∗}_{50% aller} à 23°C Pour le retour ; on enregistre la valeur maximale du facteur perte dynamique tan(δ) observée, notée tan(δ)ₘₐₓ à 23°C.

Les résultats sont indiqués en base 100 ; la valeur arbitraire 100 étant attribuée au témoin pour calculer et comparer ensuite tan(δ)ₘₐₓ à 23°C et G^{∗}_{50%aller} à 23°C des différents échantillons testés.

Pour tan(δ)ₘₐₓ à 23°C: la valeur en base 100 pour l'échantillon à tester est calculée selon l'opération : (valeur de tan(δ)ₘₐₓ à 23°C de l'échantillon à tester/valeur de tan(δ)ₘₐₓ à 23°C du témoin) × 100. De cette façon, un résultat inférieur à 100 indique une diminution de l'hystérèse qui correspond à une amélioration de la performance de résistance au roulement.

Pour G^{∗}_{50% aller} à 23°C : la valeur en base 100 pour l'échantillon à tester est calculée selon l'opération : (valeur de G^{∗}_{50% aller} à 23°C de l'échantillon à tester/valeur de G^{∗}_{50% aller} à 23°C du témoin) × 100. De cette façon, un résultat supérieur à 100 indique une amélioration du module complexe de cisaillement dynamique G^{∗}ₐₗₗₑᵣ à 50% de déformation à 23°C, qui corrobore d'une amélioration de la rigidité du matériau.

### 2 Synthèse des composés

### 2.1 - Synthèse du composé A : l'oxyde de 2-(2-(3-chloro-2-oxoimidazolidin-1-yl)éthoxy)-1- naphthonitrile

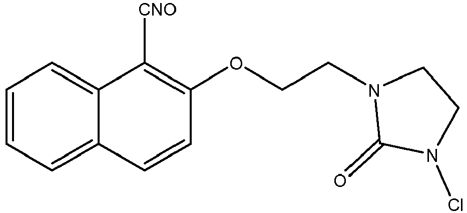

L'oxyde de 2-(2-(3-chloro-2-oxoimidazolidin-1-yl)éthoxy)-1-naphthonitrile est synthétisé en six étapes, nommées étape a1, étape a2, étape b1, étape b2, étape c et étape d.

L'étape a1 est réalisée selon le protocole 1, l'étape a2 selon le protocole 2, l'étape b1 selon le protocole 3. Les étapes b2 et c sont réalisées selon le protocole 4. L'étape d est réalisée selon le protocole 5.

### Étape a1 : préparation du 2-hydroxy-1-naphthaldéhyde

### Protocole 1 :

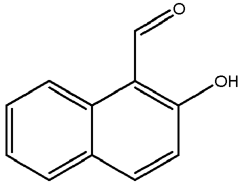

Ce composé peut être obtenu à partir du naphthol par une réaction de formylation selon le protocole dit de Reimer-Tiemann décrit dans Organic Synthèses, 22, 63-4; 1942 par Russell, Alfred et Lockhart, Luther B. ou dans Organic Reactions (Hoboken, NJ, United States), 28, 1982 par Wynberg, Hans et Meijer, Egbert W., ou selon la procédure décrite par Casiraghi, Giovanni et al. dans Journal of the Chemical Society, Perkin Transactions 1: Organic and Bio-Organic Chemistry (1972-1999), (9), 1862-5, 1980 ou encore par une réaction de Vilsmeier décrite par Jones, Gurnos et Stanforth, Stephen P. dans Organic Reactions (Hoboken, NJ, United States), 49, 1997.

Le 2-hydroxy-1-napthaldéhyde est commercial. Il peut par exemple être obtenu chez Aldrich (CAS 708-06-5).

### Étape a2 : préparation de la 1-(2-chloroéthyl)imidazolidin-2-one

### Protocole 2 :

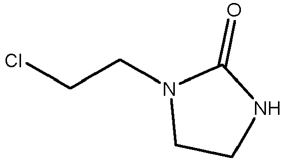

Ce composé peut être obtenu selon un protocole décrit dans la demande de brevet WO 2012/007684.

### Étape b1 : préparation du 2-(2-(2-oxoimidazolidin-1-yl)éthoxy)-1-naphthaldéhyde

### Protocole 3 :

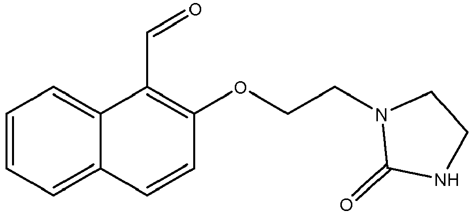

Un mélange de 2-hydroxy-1-naphthaldéhyde (20,0 g ; 0,116 mol), de carbonate de potassium (24,08 g ; 0,174 mol), et de 1-(2-chloroéthyl)imidazolidin-2-one (25,9 g ; 0,174 mol) dans le DMF (20 mL) est chauffé à 75°C (température du bain) pendant 3,0-3,5 heures. Ensuite, une seconde portion de 1-(2-chloroéthyl)imidazolidin-2-one (17,26 g ; 0,116 mol) est ajoutée et le milieu réactionnel est agité pendant 9 à 10 heures à 75°C (température du bain). Après un retour à 40-45°C, le milieu réactionnel est versé dans une solution d'hydroxyde de sodium (10 g ; 0,25 mol) dans l'eau (700 mL). Le mélange est agité pendant 10 à 15 minutes. Le précipité est filtré et lavé sur le filtre par l'eau distillée (3 fois 400 mL).

Un solide gris (31,51 g ; rendement de 95 %) est obtenu.

La pureté molaire est supérieure à 85 % (RMN ¹H).

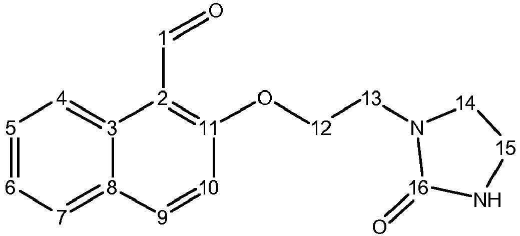

| N° | δ¹H (ppm) | δ¹³C (ppm) |
|---|---|---|
| 1 | 10,86 | 191,6 |
| 2 | / | 116,7 |
| 3 | / | 131,5 |
| 4 | 9,19 | 124,9 |
| 5 | 7,56 | 130 |
| 6 | 7,37 | 124,9 |
| 7 | 7,71 | 128,3 |
| 8 | / | 128,6 |
| 9 | 7,99 | 137,7 |
| 10 | 7,2 | 113,1 |
| 11 | / | 162,9 |
| 12 | 4,31 | 68,7 |
| 13 | 3,63 | 13,4 |
| 14 | 3,59 | 16,8 |
| 15 | 3,72 | 38,3 |
| 16 | / | 162,6 |

### Solvant : CDCl₃

### Étapes b2 et c : préparation de l'oxime de 2-(2-(2-oxoimidazolidin-1-yl)éthoxy)-1-naphthaldéhyde

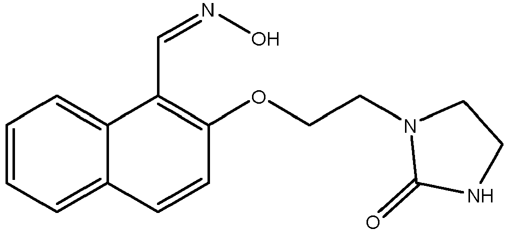

Le produit isolé à l'issue du protocole 3 est utilisé dans le protocole 4 suivant.

### Protocole 4 :

A une solution de 2-(2-(2-oxoimidazolidin-1-yl)éthoxy)-1-naphthaldéhyde (6,3 g ; 22,2 mmol) dans l'éthanol (30 mL) à 45°C (température du bain), est ajoutée une solution d'hydroxylamine (2,05 g ; 31,0 mmol, 50 % dans l'eau, Aldrich) dans l'éthanol (5 mL). Le milieu réactionnel est agité pendant 4 heures à 55 °C (température du bain). Après un retour à 40°C, de l'acétate d'éthyle (30 mL) est ajouté goutte à goutte pendant 15 minutes. Le précipité est filtré, lavé sur le filtre par un mélange éthanol/acétate d'éthyle.

Un solide blanc (4,00 g ; rendement de 60 %) est obtenu.

La pureté molaire estimée par RMN ¹H est supérieure à 95 %.

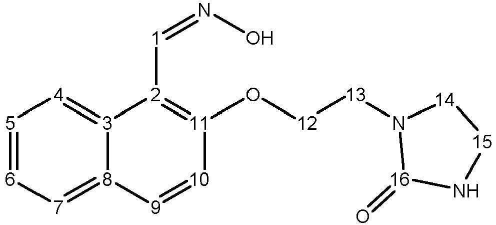

| N° | δ¹H (ppm) | δ¹³C (ppm) |
|---|---|---|
| 1 | 8,75 | 147,5 |
| 2 | / | 115,9 |
| 3 | / | 133 |
| 4 | 8,78 | 127 |
| 5 | 7,46 | 128,5 |
| 6 | 7,35 | 125,2 |
| 7 | 7,88 | 132,8 |
| 8 | / | 130,9 |
| 9 | 7,79 | 129,4 |
| 10 | 7,36 | 115,2 |
| 11 | / | 157,1 |
| 12 | 4,28 | 69,3 |
| 13 | 3,59 | 44,4 |
| 14 | 3,64 | 47,6 |
| 15 | 3,40 | 39,3 |
| 16 | / | 165,3 |

### Solvant MeOH

### Étape d : préparation de l'oxyde de 2-(2-(3-chloro-2-oxoimidazolidin-1-yl)éthoxy)-1-naphthonitrile

### Protocole 5 :

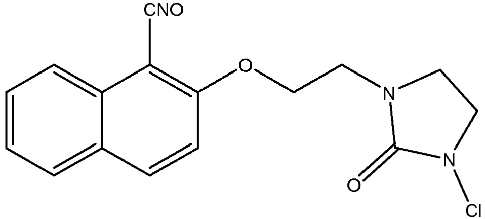

A une suspension de l'oxime de 2-(2-(2-oxoimidazolidin-1-yl)éthoxy)-1-naphthaldéhyde précédente (7,30 g ; 24,4 mmol) dans l'acétate d'éthyle (230 mL) refroidi à 1°C (température bain = 0°C) est ajouté au goutte à goutte une solution aqueuse de NaOCl (74,44 g/L ; 98 mL ; 4,0 eq. (eq=équivalent molaire) pendant 10 minutes. Le milieu réactionnel est agité pendant 8,5 heures à 0-2°C (température bain = 0°C).

Une deuxième portion de solution aqueuse de NaOCl (74,44 g/L ; 98 mL ; 4,0 eq.) est ajoutée pendant 5 minutes. Le milieu réactionnel est agité pendant 10-11 heures à 0-2°C (température bain = 0°C).

Le précipité est filtré et lavé sur filtre par l'eau (2 fois 25 mL) et puis par de l'acétate d'éthyle (2 fois 10 mL).

Un solide blanc de point de fusion 127,0-127,5 °C est alors obtenu (6,24 g ; 18,7 mmol, rendement 77 %).

Le composé A, l'oxyde de 2-(2-(3-chloro-2-oxoimidazolidin-1-yl)éthoxy)-1-naphthonitrile, est obtenu avec une pureté molaire est supérieure à 92 % (RMN ¹H) et moins de 6 % molaire d'oxyde de 2-(2-(2-oxoimidazolidin-1-yl)éthoxy)-1-naphthonitrile et 2 % molaire d'impureté de solvant résiduel.

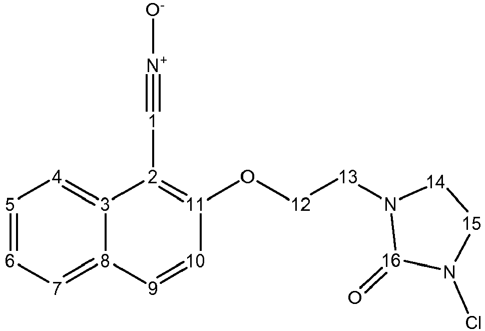

| N° | δ¹H (ppm) | δ¹³C (ppm) |
|---|---|---|
| 1 | / | / |
| 2 | / | 96,9 |
| 3 | / | 134,0 |
| 4 | 7,93 | 124,1 |
| 5 | 7,57 | 129,1 |
| 6 | 7,40 | 125,3 |
| 7 | 7,78 | 128,7 |
| 8 | / | 128,7 |
| 9 | 7,90 | 133,1 |
| 10 | 7,16 | 112,6 |
| 11 | / | 160,2 |
| 12 | 4,33 | 68,8 |
| 13 | 3,71 | 44,8 |
| 14 | 3,75 | 45,2 |
| 15 | 3,58 | 52,1 |
| 16 | / | 161,3 |

### Solvant CDCl₃

### 2.2 - Synthèse du composé B: l'oxyde 2-(2-(2-oxoimidazolidin-1-yl)éthoxy)-1-naphthonitrile

L'oxyde de 2-(2-(2-oxoimidazolidin-1-yl)éthoxy)-1-naphthonitrile est obtenu en reproduisant les étapes des protocoles 1 à 4 du paragraphe 2.1 pour obtenir l'oxime de 2-(2-(2-oxoimidazolidin-1-yl)éthoxy)-1-naphthaldéhyde, puis en effectuant le protocole 6 décrit ci-dessous.

### Protocole 6 :

A une solution d'oxime de 2-(2-(2-oxoimidazolidin-1-yl)éthoxy)-1-naphthaldéhyde (7,5 g ; 25,06 mmol) dans le dichlorométhane (300 mL) refroidi à 4°C (température bain = 0°C), est ajoutée, goutte à goutte, une solution aqueuse de NaOCl (78 g/l; 35 mL ; 1,46 eq) pendant 15 minutes. Le milieu réactionnel est agité pendant 60 à 70 minutes à une température de 4-5°C. La phase organique est séparée. La phase aqueuse est extraite par le dichlorométhane (25 mL). Les solutions organiques réunies sont lavées par l'eau (2 fois par 25 mL). Le solvant est évaporé sous pression réduite jusqu'à 40-50 mL. De l'éther de pétrole 40/60 (60 mL) est ajouté pour la cristallisation. Le précipité obtenu est filtré et lavé par l'éther de pétrole 40/60 (2 fois par 30 mL).

Un solide blanc (6,46 g ; rendement de 87 %) est obtenu.

Le produit B est obtenu avec une pureté molaire estimée par RMN ¹H est supérieure à 99 % et moins de 1 % d'impuretés de solvant résiduel.

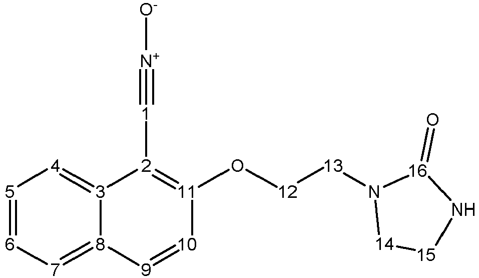

| N° | δ¹H (ppm) | δ¹³C (ppm) |
|---|---|---|
| 1 | / | / |
| 2 | / | 96,7 |
| 3 | / | 134,1 |
| 4 | 7,92 | 124 |
| 5 | 7,56 | 128,9 |
| 6 | 7,39 | 125,2 |
| 7 | 7,77 | 128,6 |
| 8 | / | 128,5 |
| 9 | 7,89 | 133 |
| 10 | 7,18 | 112,7 |
| 11 | / | 160,5 |
| 12 | 4,31 | 69,4 |
| 13 | 3,64 | 43,4 |
| 14 | 3,76 | 47,3 |
| 15 | 3,42 | 38,5 |
| 16 | / | 162,5 |

### Solvant : CDCl₃

### 3 Exemple de polymères modifiés

### 3.1 Copolymère styrène-butadiène greffé par de l'oxyde de 2-(2-(3-chloro-2-oxoimidazolidin-1-yl)éthoxy)-1-naphthonitrile

On incorpore de l'oxyde de 2-(2-(3-chloro-2-oxoimidazolidin-1-yl)éthoxy)-1-naphthonitrile (0,27 g; 0,81 mmol), composé A obtenu par le procédé de synthèse décrit au paragraphe 2.1, à 20 g de copolymère styrène-butadiène non-fonctionnalisé (contenant 26,5 % en poids de styrène par rapport au poids total du polymère et 25 % en poids d'unité butadiène-1,2, par rapport au poids de la partie butadiènique ; Mn = 150000 g/mol et Ip=1,84 mesurés selon la méthode décrite au paragraphe 1.2) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,29 % avec un rendement molaire de greffage de 96 %.

Déplacement chimique permettant la détermination du taux de greffage :
7.6 à 8.2 ppm : 6 H aromatiques
4.2 4.3 ppm : 2H CH₂-O
3.1-3.8 ppm: 6H CH₂-N

### 3.2 Polyisoprène greffé par de l'oxyde de 2-(2-(3-chloro-2-oxoimidazolidin-1-yl)éthoxy)-1-naphthonitrile

On incorpore l'oxyde de 2-(2-(3-chloro-2-oxoimidazolidin-1-yl)éthoxy)-1-naphthonitrile (0,29 g; 0,88 mmol), composé A obtenu par le procédé de synthèse décrit au paragraphe 1.2, à 20 g d'un polyisoprène synthétique (contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,2 % avec un rendement molaire de greffage de 66 %.

Déplacement chimique permettant la détermination du taux de greffage :
7.6 à 8.2 ppm : 6 H aromatiques
4.2 4.3 ppm : 2H CH₂-O
3.1-3.8 ppm : 6H CH₂-N

### 4 Exemple de compositions de caoutchouc

### 4.1 Exemple 1

Cet essai a pour objet de montrer l'amélioration de performance en termes de propriétés de caoutchouterie d'une composition de l'invention par rapport à une composition non conforme classique ne contenant pas d'élastomère modifié et à une composition de l'art antérieur comprenant un élastomère modifié. En particulier, ces compositions sont définies de la façon suivante :
- la composition non-conforme C1 est une composition « classique » de caoutchouc utilisée comme bande de roulement pour pneumatique, incluant notamment un polyisoprène de synthèse non greffé, de la silice et un agent de couplage polysulfure silane ;
- la composition non conforme C2 est identique à la composition C1 à l'exception qu'elle comprend en plus le composé B qui se greffe sur le polyisoprène de synthèse ;
- la composition C3, conforme à l'invention, est une composition identique à la composition C1 à l'exception qu'elle comprend en plus le composé A qui se greffe sur le polyisoprène de synthèse.

Le taux des différents constituants de ces compositions exprimés en pce, partie en poids pour cent parties en poids d'élastomère, sont présentés dans le tableau 1 :

**Tableau 1**

| | **C1** | **C2** | **C3** |
|---|---|---|---|
| Élastomère diénique (1) | 100 | 100 | 100 |
| Composé B (2) | | 1,31 | |
| Composé A (3) | | | 1,46 |
| Charge renforçante (4) | 60 | 60 | 60 |
| Agent de couplage (5) | 6 | 6 | 6 |
| Noir de carbone (6) | 3 | 3 | 3 |
| Antioxydant (7) | 1,5 | 1,5 | 1,5 |
| TMQ (8) | 1 | 1 | 1 |
| Paraffine | 1 | 1 | 1 |
| ZnO (9) | 2,7 | 2,7 | 2,7 |
| Acide stéarique (10) | 2,5 | 2,5 | 2,5 |
| CBS (11) | 1,6 | 1,6 | 1,6 |
| Soufre | 1,3 | 1,3 | 1,3 |

Les compositions C2 et C3 comprennent le même nombre de mol de composé A ou B greffé, à savoir 0,2 % molaire.
(1) Polyisoprène contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2 ;
(2) Composé B : oxyde de 2-(2-(2-oxoimidazolidin-1-yl)éthoxy)-1-naphthonitrile synthétisé selon le procédé décrit au paragraphe 2.2 ;
(3) Composé A : oxyde de 2-(2-(3-chloro-2-oxoimidazolidin-1-yl)éthoxy)-1-naphthonitrile synthétisé selon le procédé décrit au paragraphe 2.1 ;
(4) Silice « Zeosil 1165MP » commercialisée par Solvay ;
(5) Bis[3-(triéthoxysilyl)propyl] tétrasulfure silane (TESPT) commercialisé par Evonik sous la référence « Si69 » ;
(6) Noir de carbone de grade N234 commercialisé par Cabot Corporation ;
(7) N-1,3-diméthylbutyl-N-phenyl-para-phénylènediamine commercialisé par Flexys sous la référence « Santoflex 6-PPD » ;
(8) 2,2,4-triméthyl-1,2-dihydroquinoline commercialisée par la société Flexys ;
(9) Oxyde de Zinc (grade industriel) commercialisé par la société Umicore ;
(10) Stéarine « Pristerene 4031 » commercialisée par la société Uniquema ;
(11) N-cyclohexyl-2-benzothiazyl-sulfénamide commercialisé par Flexys sous la référence « Santocure CBS ».

Les compositions C1 à C3 sont préparées de la manière suivante : on introduit dans un mélangeur interne « Polylab » de 85 cm³, rempli à 70 % et dont la température initiale de cuve est d'environ 110°C, l'élastomère diénique. Pour les compositions concernant le composé A ou le composé B, le composé A ou le composé B est introduit en même temps que l'élastomère diénique. Puis un travail thermomécanique d'une minute et demie est conduit à une température de 120°C.

Ensuite, pour chacune des compositions C1 à C3, on introduit la ou les charges renforçantes, l'agent de couplage de la charge avec l'élastomère diénique, puis après une à deux minutes de malaxage, les divers autres ingrédients à l'exception du système de vulcanisation. On conduit alors un travail thermomécanique (phase non-productive) en une étape, qui dure au total environ 5 minutes, jusqu'à atteindre une température maximale de tombée de 160°C.

On récupère le mélange ainsi obtenu, on le refroidit puis on ajoute le système de vulcanisation (soufre et l'accélérateur type sulfénamide) sur un mélangeur externe (homo-finisseur) à 25°C, en mélangeant l'ensemble (phase productive) pendant environ 5 à 6 minutes.

Les compositions ainsi obtenues sont ensuite calandrées sous la forme de plaques (épaisseur de 2 à 3 mm) ou de feuilles fines de caoutchouc pour la mesure de leurs propriétés physiques ou mécaniques.

Les propriétés de caoutchouterie de ces compositions sont mesurées après cuisson à 150°C pendant 60 minutes. Les résultats obtenus sont portés dans le tableau 2.

**Tableau 2**

| Compositions | **C1** | **C2** | **C3** |
|---|---|---|---|
| Tan(δ)ₘₐₓ à 23 C° | 100 | 80 | 70 |

On constate au vu du tableau 2, de façon attendue, que la composition non conforme C2 qui comprend un élastomère modifié avec un composé comportant une fonction imidazolidinone non substituée, présente une diminution de l'hystérèse (tan(δ)ₘₐₓ à 23°C), par rapport à la composition non conforme C1 qui ne comprend pas d'élastomère modifié.

De façon surprenante, la composition C3 selon l'invention qui comprend un élastomère modifié avec un composé comportant une fonction imidazolidinone N-substituée présente une diminution significative de l'hystérèse par rapport à la composition non conforme C1 ainsi qu'une diminution significative de l'hystérèse par rapport à la composition non conforme C2 qui présentait déjà de bonnes propriétés hystérétiques par rapport à la composition non conforme C1.

L'élastomère modifié par le composé utilisé dans le cadre de la présente invention permet donc une diminution de l'hystérèse des compositions le contenant, et donc d'améliorer la performance résistance au roulement de ces compositions.

### 4.2 Exemple 2

Cet essai a pour objet de montrer l'amélioration des propriétés de caoutchouterie de plusieurs compositions selon l'invention par rapport à des compositions non conformes classiques ne comprenant pas d'élastomère modifié et à des compositions de l'art antérieur non conformes comprenant un élastomère modifié. En particulier, ces compositions sont définies de la façon suivante :
- la composition non-conforme C4 est une composition « classique » de caoutchouc utilisée comme bande de roulement pour pneumatique, incluant notamment un copolymère styrène-butadiène (SBR) non greffé, de la silice et un agent de couplage polysulfure silane ;
- la composition non conforme C5 est identique à la composition C4 à l'exception qu'elle comprend en plus le composé B qui se greffe sur le SBR ;
- La composition C6, conforme à l'invention, est une composition identique à la composition C5 à l'exception qu'elle comprend en plus les composés A et B qui se greffent sur le SBR ;
- la composition non-conforme C7 est identique à la composition C4 sauf qu'elle comprend un polyisoprène de synthèse à la place du SBR ;
- la composition non conforme C8 est identique à la composition C7 à l'exception qu'elle comprend en plus le composé B qui se greffe sur le polyisoprène de synthèse ;
- la composition C9, conforme à l'invention, est une composition identique à la composition C7 à l'exception qu'elle comprend en plus les composés A et B qui se greffent sur le polyisoprène de synthèse.

Le taux des différents constituants des compositions exprimés en pce, partie en poids pour cent parties en poids d'élastomère, sont présentés dans le tableau 3 qui suit :

**Tableau 3**

| | **C4** | **C5** | **C6** | **C7** | **C8** | **C9** |
|---|---|---|---|---|---|---|
| Élastomère diénique (1) | | | | 100 | 100 | 100 |
| Élastomère diénique (2) | 100 | 100 | 100 | | | |
| Composé B (3) | | 1,31 | 1,31 | | 1,31 | 1,31 |
| Composé A (4) | | | 0,49 | | | 0,49 |
| Charge renforçante (5) | 60 | 60 | 60 | 60 | 60 | 60 |
| Agent de couplage (6) | 6 | 6 | 6 | 6 | 6 | 6 |
| Noir de carbone (7) | 3 | 3 | 3 | 3 | 3 | 3 |
| Antioxydant (8) | 1,5 | 1,5 | 1,5 | 1,5 | 1,5 | 1,5 |
| TMQ (9) | 1 | 1 | 1 | 1 | 1 | 1 |
| Paraffine | 1 | 1 | 1 | 1 | 1 | 1 |
| ZnO (10) | 2,7 | 2,7 | 2,7 | 2,7 | 2,7 | 2,7 |
| Acide stéarique (11) | 2,5 | 2,5 | 2,5 | 2,5 | 2,5 | 2,5 |
| CBS (12) | 1,6 | 1,6 | 1,6 | 1,6 | 1,6 | 1,6 |
| Soufre | 1,3 | 1,3 | 1,3 | 1,3 | 1,3 | 1,3 |

(1) Polyisoprène contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2 ;
(2) Copolymère de styrène-butadiène non-fonctionnalisé contenant 26,5% en poids de styrène par rapport au poids total du copolymère et 25 % en poids d'unité butadiène-1,2 par rapport au poids de la partie butadiènique ; Mn = 150000 g/mol et Ip=1,84 mesurés selon la méthode décrite au paragraphe 1.2 ;
(3) Composé B : oxyde de 2-(2-(2-oxoimidazolidin-1-yl)éthoxy)-1-naphthonitrile synthétisé selon le procédé décrit au paragraphe 2.2 ;
(4) Composé A : oxyde de 2-(2-(3-chloro-2-oxoimidazolidin-1-yl)éthoxy)-1-naphthonitrile synthétisé selon le procédé décrit au paragraphe 2.1 ;
(5) Silice « Zeosil 1165MP » commercialisée par Solvay ;
(6) Bis[3-(triéthoxysilyl)propyl] tétrasulfure silane (TESPT) commercialisé par Evonik sous la référence « Si69 » ;
(7) Noir de carbone de grade N234 commercialisé par Cabot Corporation ;
(8) N-1,3-diméthylbutyl-N-phenyl-para-phénylènediamine commercialisé par Flexys sous la référence « Santoflex 6-PPD » ;
(9) 2,2,4-triméthyl-1,2-dihydroquinoline commercialisée par la société Flexys ;
(10) Oxyde de Zinc (grade industriel) commercialisé par la société Umicore ;
(11) Stéarine « Pristerene 4031 » commercialisée par la société Uniquema ;
(12) N-cyclohexyl-2-benzothiazyl-sulfénamide commercialisé par Flexys sous la référence « Santocure CBS ».

Les compositions sont préparées de la manière suivante : on introduit dans un mélangeur interne « Polylab » de 85 cm³, rempli à 70% et dont la température initiale de cuve est d'environ 110°C, l'élastomère diénique. Pour les compositions concernant le composé B ou les composés A et B, le composé B ou les composés A et B sont introduits en même temps que l'élastomère diénique. Puis un travail thermomécanique d'une minute et demie est conduit à une température de 120°C.

Ensuite, pour chacune des compositions C4 à C9, on introduit la ou les charges renforçantes, l'agent de coupage de la charge avec l'élastomère diénique, puis après une à deux minutes de malaxage, les divers autres ingrédients à l'exception du système de vulcanisation. On conduit alors un travail thermomécanique (phase non-productive) en une étape, qui dure au total environ 5 minutes, jusqu'à atteindre une température maximale de tombée de 160°C.

On récupère le mélange ainsi obtenu, on le refroidit puis on ajoute le système de vulcanisation (soufre et l'accélérateur type sulfénamide) sur un mélangeur externe (homo-finisseur) à 25°C, en mélangeant l'ensemble (phase productive) pendant environs 5 à 6 minutes.

Les compositions ainsi obtenues sont ensuite calandrées sous la forme de plaques (épaisseur de 2 à 3 mm) ou de feuilles fines de caoutchouc pour la mesure de leurs propriétés physiques ou mécaniques. Les propriétés de caoutchouterie de ces compositions sont mesurées après cuisson à 150°C pendant 60 min. Les résultats obtenus sont portés dans le tableau 4.

**Tableau 4**

| | **C4** | **C5** | **C6** | **C7** | **C8** | **C9** |
|---|---|---|---|---|---|---|
| G ^{∗}_{50% aller} à 23°C | 100 | 100 | 99 | 100 | 100 | 93 |
| Tan (δ) ₘₐₓ à 23 C° | 100 | 81 | 76 | 100 | 80 | 65 |

On constate, au vu du tableau 4, de façon attendue, que les compositions non conforme C5 et C8 qui comprennent un élastomère greffé avec un composé comportant des fonctions imidazolidinone non-substituées, présentent une diminution de l'hystérèse à iso-rigidité (G ^{∗}_{50% aller} à 23°C) par rapport aux compositions non conformes C4 et C7 respectivement qui ne comprennent pas d'élastomère modifié.

De façon surprenante, la composition C6 selon l'invention qui comprend un élastomère modifié avec un composé portant des fonctions imidazolidinone N-substituées et un composé portant des fonctions imidazolidinone non-substituées présente une diminution de l'hystérèse significative par rapport à la composition non conforme C5 qui présentait déjà une hystérèse diminuée par rapport à la composition non conforme C4. Cette diminution de l'hystérèse s'effectue sans une diminution des propriétés de rigidité à cuit, ce qui est surprenant. En effet, l'homme du métier sait que l'hystérèse et les propriétés de rigidité à cuit sont deux propriétés antagonistes des compositions de caoutchouc. Il est connu qu'une amélioration des propriétés de rigidité à cru entraîne une dégradation de l'hystérèse et inversement. Or on observe ici une amélioration significative du compromis rigidité à cuit/hystérèse de la composition C6 conforme à l'invention, par rapport aux compositions C4 et C5 non conformes. Ce même résultat est obtenu avec la composition C9 conforme à l'invention par rapport aux compositions non conformes C7 et C8 ; même si une baisse de la rigidité à cru est observée pour la composition C9 selon l'invention par rapport à la composition C8 non conforme. Cette diminution de la rigidité à cru reste très faible compte tenu de la forte baisse de l'hystérèse de la composition C9 selon l'invention.

## Revendications

1. Composition de caoutchouc à base d'au moins un élastomère diénique, d'au moins une charge renforçante, d'au moins un système de réticulation chimique et d'au moins un composé de formule (I) éventuellement déjà greffé sur ledit élastomère diénique 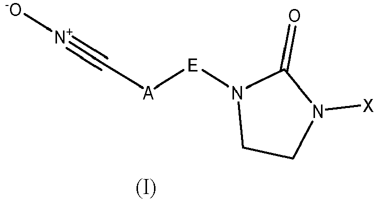 dans laquelle :
• A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
• E représente un groupe divalent hydrocarboné comprenant éventuellement un ou plusieurs hétéroatomes ; et
• X désigne un atome d'halogène, de préférence un atome de chlore.

2. Composition de caoutchouc selon la revendication 1, dans laquelle l'élastomère diénique est choisi dans le groupe constitué par les copolymères d'éthylène-propylène-monomère diène, le caoutchouc butyle, le caoutchouc naturel, les polyisoprènes de synthèse, les polybutadiènes, les copolymères de butadiène, les copolymères d'isoprène et les mélanges de ces élastomères.

3. Composition de caoutchouc selon l'une quelconque des revendications 1 à 2, dans laquelle le composé de formule (I) est choisi parmi les composés de formule (Ia) et (Ib) 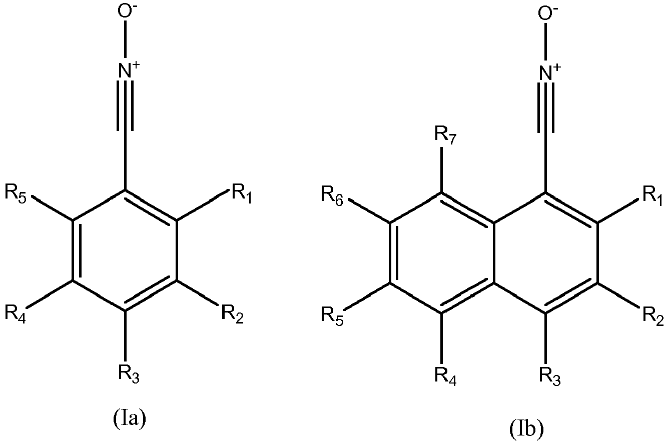 dans lesquelles :
• un groupement choisi parmi R₁ à R₅ de la formule (Ia) et un groupement choisi parmi R₁ à R₇ de la formule (Ib) désignent le groupe de formule (II) suivante : 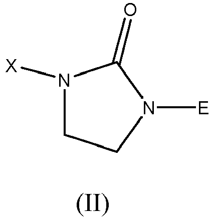 dans laquelle E et X sont tels que défini à la revendication 1 ; et
• les quatre groupements de la formule (Ia) choisis parmi R₁ à R₅ autres que celui désignant le groupe de formule (II) et les six groupements de la formule (Ib) choisis parmi R₁ à R₇ autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

4. Composition de caoutchouc selon l'une quelconque des revendications précédentes, dans laquelle le groupement E est une chaîne hydrocarbonée, linéaire ou ramifiée, de préférence saturée, en C₁-C₂₄, de préférence en C₁-C₁₀, plus préférentiellement en C₁-C₆, éventuellement interrompue par un ou plusieurs atomes d'azote, de soufre ou d'oxygène.

5. Composition de caoutchouc selon l'une quelconque des revendications 3 à 4, dans laquelle le composé de formule (I) est le composé de formule (Ib).

6. Composition de caoutchouc l'une quelconque des revendications précédentes, dans laquelle le composé de formule (I) est le composé de formule (III)

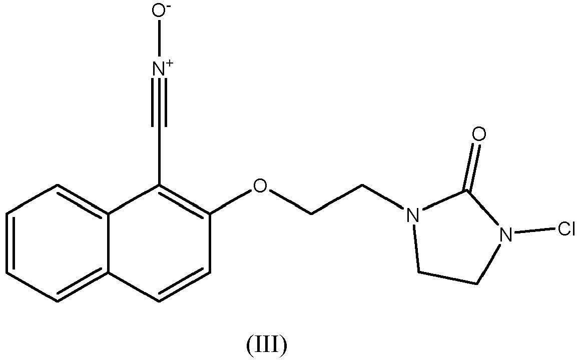

7. Composition de caoutchouc selon l'une quelconque des revendications précédentes, comprenant en outre au moins un composé de formule (V) éventuellement déjà greffé sur ledit élastomère diénique 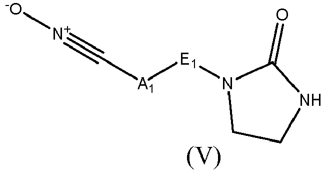 dans laquelle :
• A₁ représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
• E₁ représente un groupe divalent hydrocarboné comprenant éventuellement un ou plusieurs hétéroatomes.

8. Composition de caoutchouc selon la revendication 7, dans laquelle le composé de formule (V) est choisi parmi les composés de formule (Va) et (Vb) 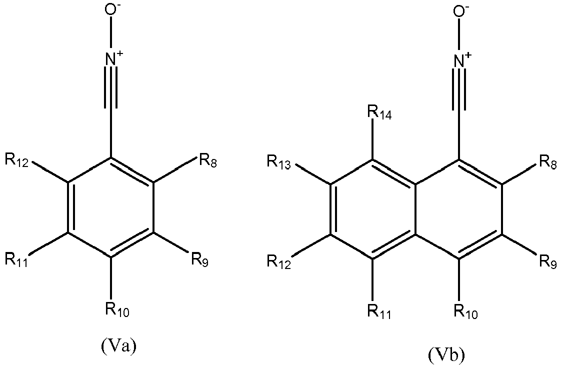 dans lesquelles :
• un groupement choisi parmi R₈ à R₁₂ de la formule (Va) et un groupement choisi parmi R₈ à R₁₄ de la formule (Vb) désignent le groupe de formule (VI) suivante : 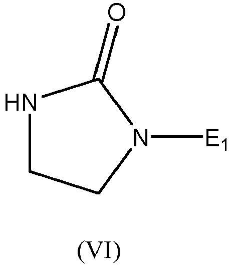 avec E₁ tel que défini à la revendication 7 ; et
• les quatre groupements de la formule (Va) choisis parmi R₈ à R₁₂ autres que celui désignant le groupe de formule (VI) et les six groupements de la formule (Vb) choisis parmi R₈ à R₁₄ autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

9. Composition de caoutchouc l'une quelconque des revendications 7 à 8, dans laquelle le groupement E₁ du composé de formule (V) est une chaîne hydrocarbonée, linéaire ou ramifiée, de préférence saturée, en C₁-C₂₄, de préférence en C₁-C₁₀, plus préférentiellement en C₁-C₆, éventuellement interrompue par un ou plusieurs atomes d'azote, de soufre ou d'oxygène.

10. Composition de caoutchouc selon l'une quelconque des revendications 8 à 9, dans laquelle le composé de formule (V) est le composé de formule (Vb).

11. Composition de caoutchouc selon l'une quelconque des revendications 7 à 10, dans laquelle le composé de formule (V) est le composé de formule (VII)

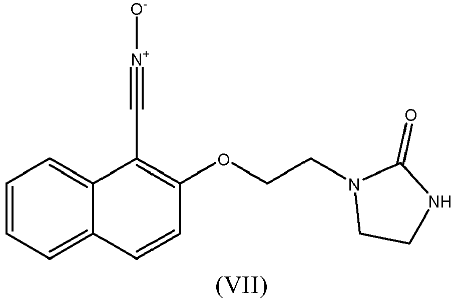

12. Composition de caoutchouc selon l'une quelconque des revendications précédentes, dans laquelle la charge renforçante est choisie parmi un noir de carbone, une charge inorganique renforçante et un mélange d'un noir de carbone et d'une charge inorganique.

13. Compositions de caoutchouc selon l'une quelconque des revendications précédentes comprenant en outre un plastifiant.

14. Article semi-fini en caoutchouc pour pneumatique, **caractérisé en ce qu'**il comprend au moins une composition de caoutchouc telle que définie selon l'une quelconque des revendications 1 à 13.

15. Pneumatique, **caractérisé en ce qu'**il comporte au moins un article semi-fini selon la revendication 14 ou au moins une composition selon l'une quelconque des revendications 1 à 13.

## Patentansprüche

1. Kautschukzusammensetzung auf Basis von mindestens einem Dienelastomer, mindestens einem verstärkenden Füllstoff, mindestens einem chemischen Vernetzungssystem und mindestens einer gegebenenfalls bereits auf das Dienelastomer aufgepfropften Verbindung der Formel (I): 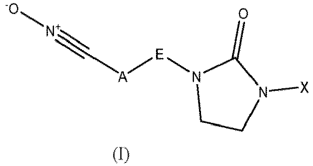 in der:
• A für einen Arendiylring steht, der gegebenenfalls durch eine oder mehrere gleiche oder verschiedene, aliphatische Kohlenwasserstoffketten substituiert ist, die vorzugsweise gesättigt sind, linear oder verzweigt sind und gegebenenfalls substituiert oder durch ein oder mehrere Heteroatome unterbrochen sind;
• E für eine zweiwertige Kohlenwasserstoffgruppe steht, die gegebenenfalls ein oder mehrere Heteroatome enthalten kann; und
• X ein Halogenatom, vorzugsweise ein Chloratom, bedeutet.

2. Kautschukzusammensetzung nach Anspruch 1, wobei das Dienelastomer aus der Gruppe bestehend aus Ethylen-Propylen-Dienmonomer-Copolymeren, Butylkautschuk, Naturkautschuk, synthetischen Polyisoprenen, Polybutadienen, Butadien-Copolymeren, Isopren-Copolymeren und Mischungen dieser Elastomere ausgewählt ist.

3. Kautschukzusammensetzung nach einem der Ansprüche 1 bis 2, wobei die Verbindung der Formel (I) aus den Verbindungen der Formel (Ia) und (Ib) ausgewählt ist: 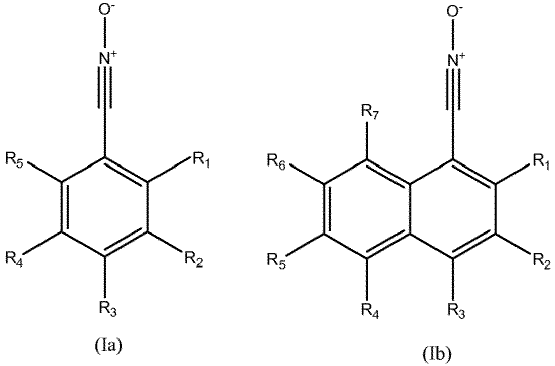 in denen:
• eine aus R₁ bis R₅ der Formel (Ia) ausgewählte Gruppe und eine aus R₁ bis R₇ der Formel (Ib) ausgewählte Gruppe die Gruppe der folgenden Formel (II) bedeuten: 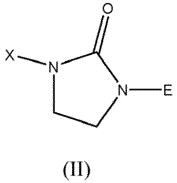 in der E und X wie in Anspruch 1 definiert sind; und
• die vier aus R₁ bis R₅ der Formel (Ia) ausgewählten Gruppen, die keine Gruppe der Formel (II) bedeuten, und die sechs aus R₁ bis R₇ der Formel (Ib) ausgewählten Gruppen, die keine Gruppe der Verbindung (II) bedeuten, gleich oder verschieden sind und unabhängig voneinander für ein Wasserstoffatom oder eine aliphatische Kohlenwasserstoffkette, die vorzugsweise gesättigt ist, linear oder verzweigt ist und gegebenenfalls substituiert oder durch ein oder mehrere Heteroatome unterbrochen ist, stehen.

4. Kautschukzusammensetzung nach einem der vorhergehenden Ansprüche, wobei es sich bei der Gruppe E um eine lineare oder verzweigte, vorzugsweise gesättigte C₁-C₂₄-, vorzugsweise C₁-C₁₀- und weiter bevorzugt C₁-C₆-Kohlenwasserstoffkette, die gegebenenfalls durch ein oder mehrere Stickstoff-, Schwefel- oder Sauerstoffatome unterbrochen ist, handelt.

5. Kautschukzusammensetzung nach einem der Ansprüche 3 bis 4, wobei es sich bei der Verbindung der Formel (I) um eine Verbindung der Formel (Ib) handelt.

6. Kautschukzusammensetzung nach einem der vorhergehenden Ansprüche, wobei es sich bei der Verbindung der Formel (I) um die Verbindung der Formel (III) handelt:

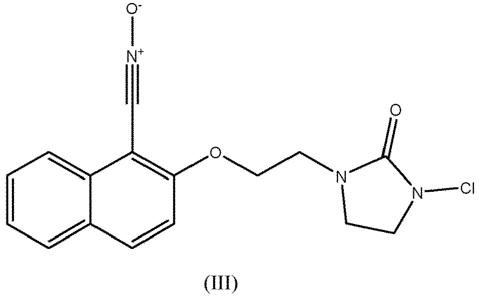

7. Kautschukzusammensetzung nach einem der vorhergehenden Ansprüche, die außerdem eine gegebenenfalls bereits auf das Dienelastomer aufgepfropfte Verbindung der Formel (V) umfasst: 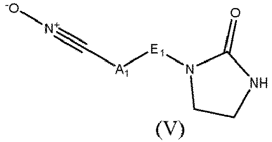 in der:
• A₁ für einen Arendiylring steht, der gegebenenfalls durch eine oder mehrere gleiche oder verschiedene, aliphatische Kohlenwasserstoffketten substituiert ist, die vorzugsweise gesättigt sind, linear oder verzweigt sind und gegebenenfalls substituiert oder durch ein oder mehrere Heteroatome unterbrochen sind; und
• E₁ für eine zweiwertige Kohlenwasserstoffgruppe steht, die gegebenenfalls ein oder mehrere Heteroatome enthalten kann.

8. Kautschukzusammensetzung nach Anspruch 7, wobei die Verbindung der Formel (V) aus den Verbindungen der Formel (Va) und (Vb) ausgewählt ist: 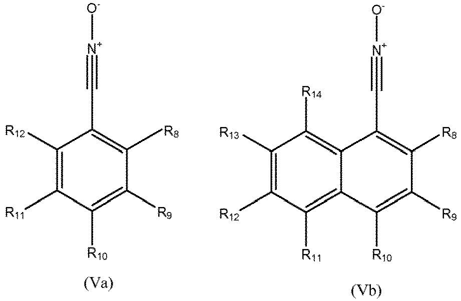 in denen:
• eine aus R₈ bis R₁₂ der Formel (Va) ausgewählte Gruppe und eine aus R₈ bis R₁₄ der Formel (Vb) ausgewählte Gruppe die Gruppe der folgenden Formel (VI) bedeuten: 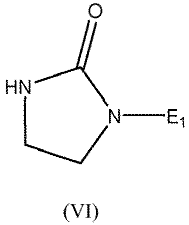 Wobei E₁ wie in Anspruch 7 definiert ist; und
• die vier aus R₈ bis R₁₂ der Formel (Va) ausgewählten Gruppen, die keine Gruppe der Formel (VI) bedeuten, und die sechs aus R₈ bis R₁₄ der Formel (Vb) ausgewählten Gruppen, die keine Gruppe der Verbindung (VI) bedeuten, gleich oder verschieden sind und unabhängig voneinander für ein Wasserstoffatom oder eine aliphatische Kohlenwasserstoffkette, die vorzugsweise gesättigt ist, linear oder verzweigt ist und gegebenenfalls substituiert oder durch ein oder mehrere Heteroatome unterbrochen ist, stehen.

9. Kautschukzusammensetzung nach einem der Ansprüche 7 bis 8, wobei es sich bei der Gruppe E₁ um eine lineare oder verzweigte, vorzugsweise gesättigte C₁-C₂₄-, vorzugsweise C₁-C₁₀- und weiter bevorzugt C₁-C₆-Kohlenwasserstoffkette, die gegebenenfalls durch ein oder mehrere Stickstoff-, Schwefel- oder Sauerstoffatome unterbrochen ist, handelt.

10. Kautschukzusammensetzung nach einem der Ansprüche 8 bis 9, wobei es sich bei der Verbindung der Formel (V) um die Verbindung der Formel (Vb) handelt.

11. Kautschukzusammensetzung nach einem der Ansprüche 7 bis 10, wobei es sich bei der Verbindung der Formel (V) um die Verbindung der Formel (VII) handelt:

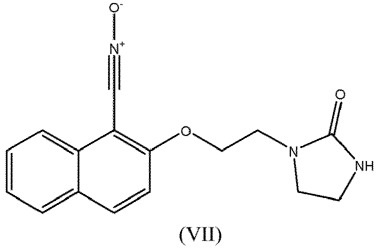

12. Kautschukzusammensetzung nach einem der vorhergehenden Ansprüche, wobei der verstärkende Füllstoff aus einem Ruß, einem verstärkenden anorganischen Füllstoff und einer Mischung von Ruß und einem anorganischen Füllstoff ausgewählt ist.

13. Kautschukzusammensetzungen nach einem der vorhergehenden Ansprüche, die außerdem einen Weichmacher umfassen.

14. Kautschukhalbzeug für Reifen, **dadurch gekennzeichnet, dass** es mindestens eine Kautschukzusammensetzung gemäß einem der Ansprüche 1 bis 13 umfasst.

15. Reifen, **dadurch gekennzeichnet, dass** er mindestens ein Halbzeug nach Anspruch 14 oder mindestens eine Zusammensetzung nach einem der Ansprüche 1 bis 13 umfasst.

## Claims

1. Rubber composition based on at least one diene elastomer, on at least one reinforcing filler, on at least one chemical crosslinking system and on at least one compound of formula (I) optionally pre-grafted to said diene elastomer: 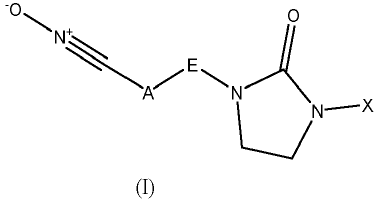 in which:
• A represents an arenediyl ring optionally substituted by one or more identical or different, preferably saturated, linear or branched, aliphatic hydrocarbon chains, which are optionally substituted or interrupted by one or more heteroatoms;
• E represents a divalent hydrocarbon group optionally comprising one or more heteroatoms; and
• X denotes a halogen atom, preferably a chlorine atom.

2. Rubber composition according to Claim 1, in which the diene elastomer is selected from the group consisting of ethylene/propylene/diene monomer copolymers, butyl rubber, natural rubber, synthetic polyisoprenes, polybutadienes, butadiene copolymers, isoprene copolymers and the mixtures of these elastomers.

3. Rubber composition according to either one of Claims 1 and 2, in which the compound of formula (I) is chosen from the compounds of formula (Ia) and (Ib): 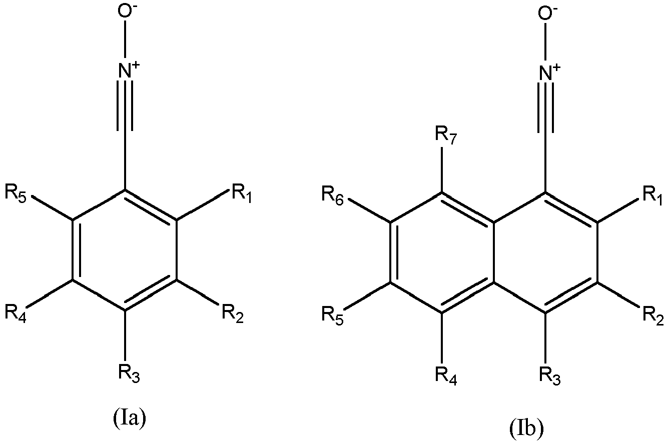 in which:
• a group chosen from R₁ to R₅ of the formula (Ia) and a group chosen from R₁ to R₇ of the formula (Ib) denote the group of following formula (II): 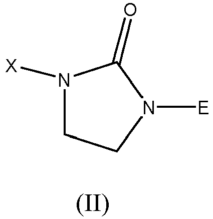 in which E and X are as defined in Claim 1; and
• the four groups of the formula (Ia) chosen from R₁ to R₅ other than that denoting the group of formula (II) and the six groups of the formula (Ib) chosen from R₁ to R₇ other than that denoting the group of formula (II), which are identical or different, represent, independently of one another, a hydrogen atom or a, preferably saturated, linear or branched aliphatic hydrocarbon chain optionally substituted or interrupted by one or more heteroatoms.

4. Rubber composition according to any one of the preceding claims, in which the group E is a, preferably saturated, linear or branched C₁-C₂₄, preferably C₁-C₁₀, more preferentially C₁-C₆, hydrocarbon chain optionally interrupted by one or more nitrogen, sulfur or oxygen atoms.

5. Rubber composition according to either one of Claims 3 and 4, in which the compound of formula (I) is the compound of formula (Ib).

6. Rubber composition any one of the preceding claims, in which the compound of formula (I) is the compound of formula (III):

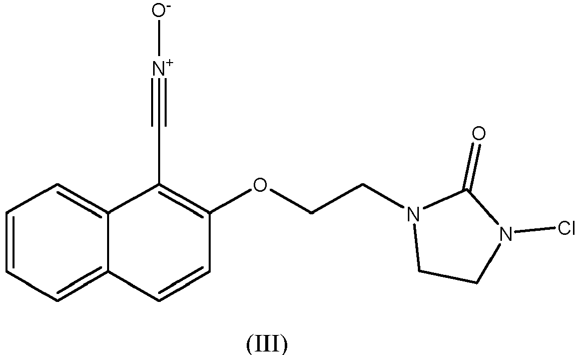

7. Rubber composition according to any one of the preceding claims, additionally comprising at least one compound of formula (V), optionally pre-grafted to said diene elastomer: 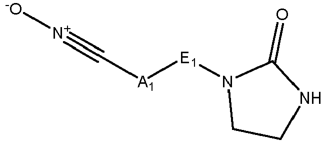 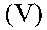 in which:
• A₁ represents an arenediyl ring optionally substituted by one or more identical or different, preferably saturated, linear or branched aliphatic hydrocarbon chains, which are optionally substituted or interrupted by one or more heteroatoms; and
• E₁ represents a divalent hydrocarbon group optionally comprising one or more heteroatoms.

8. Rubber composition according to Claim 7, in which the compound of formula (V) is chosen from the compounds of formula (Va) and (Vb): 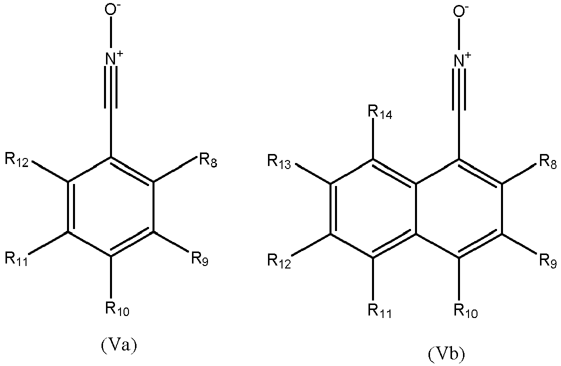 in which:
• a group chosen from R₈ to R₁₂ of the formula (Va) and a group chosen from R₈ to R₁₄ of the formula (Vb) denote the group of following formula (VI): 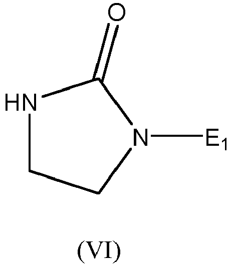 with E₁ as defined in Claim 7; and
• the four groups of the formula (Va) chosen from R₈ to R₁₂ other than that denoting the group of formula (VI) and the six groups of the formula (Vb) chosen from R₈ to R₁₄ other than that denoting the group of formula (VI), which are identical or different, represent, independently of one another, a hydrogen atom or a, preferably saturated, linear or branched aliphatic hydrocarbon chain optionally substituted or interrupted by one or more heteroatoms.

9. Rubber composition either one of Claims 7 and 8, in which the group E₁ of the compound of formula (V) is a, preferably saturated, linear or branched C₁-C₂₄, preferably C₁-C₁₀, more preferentially C₁-C₆, hydrocarbon chain optionally interrupted by one or more nitrogen, sulfur or oxygen atoms.

10. Rubber composition according to either one of Claims 8 and 9, in which the compound of formula (V) is the compound of formula (Vb).

11. Rubber composition according to any one of Claims 7 to 10, in which the compound of formula (V) is the compound of formula (VII):

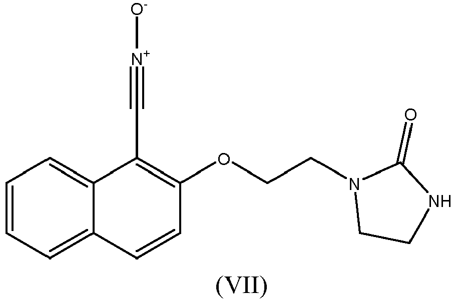

12. Rubber composition according to any one of the preceding claims, in which the reinforcing filler is chosen from a carbon black, a reinforcing inorganic filler and a mixture of a carbon black and of an inorganic filler.

13. Rubber compositions according to any one of the preceding claims, additionally comprising a plasticizer.

14. Semi-finished article made of rubber for a tyre, **characterized in that** it comprises at least one rubber composition as defined according to any one of Claims 1 to 13.

15. Tyre, **characterized in that** it comprises at least one semi-finished article according to Claim 14 or at least one composition according to any one of Claims 1 to 13.
